(19) Europäisches Patentamt
European Patent Office
Office européen des brevets

(11) **EP 1 173 452 B1**

(12) # EUROPÄISCHE PATENTSCHRIFT

(45) Veröffentlichungstag und Bekanntmachung des Hinweises auf die Patenterteilung:
**23.04.2003 Patentblatt 2003/17**

(21) Anmeldenummer: **00927137.0**

(22) Anmeldetag: **04.05.2000**

(51) Int Cl.⁷: **C07F 17/00**, C07F 17/02, C07C 249/02, C08F 10/00

(86) Internationale Anmeldenummer:
**PCT/EP00/04005**

(87) Internationale Veröffentlichungsnummer:
**WO 00/066600 (09.11.2000 Gazette 2000/45)**

(54) **METALLORGANISCHE KATALYSATOREN FÜR DIE POLYMERISATION UNGESÄTTIGTER VERBINDUNGEN**

METAL ORGANIC CATALYSTS FOR POLYMERIZING UNSATURATED COMPOUNDS

CATALYSEURS ORGANOMETALLIQUES POUR POLYMERISER DES COMPOSES INSATURES

(84) Benannte Vertragsstaaten:
**AT BE CH CY DE DK ES FI FR GB GR IE IT LI LU MC NL PT SE**

(30) Priorität: **04.05.1999 DE 19920486**

(43) Veröffentlichungstag der Anmeldung:
**23.01.2002 Patentblatt 2002/04**

(73) Patentinhaber: **BASF AKTIENGESELLSCHAFT**
**67056 Ludwigshafen (DE)**

(72) Erfinder:
- **GONIOUKH, Andrei**
  **D-67373 Dudenhofen (DE)**
- **MICKLITZ, Wolfgang**
  **D-67434 Neustadt (DE)**
- **BILDSTEIN, Benno**
  **A-6020 Innsbruck (AT)**
- **MALAUN, Michael**
  **A-6473 Wenns (AT)**
- **HRADSKY, Andreas**
  **A-6020 Innsbruck (AT)**

(56) Entgegenhaltungen:
**GB-A- 2 314 518**

- **BILDSTEIN, B. ET AL.: "n,n-diferrocenyl-n-heterocyclic carbenes and their derivatives" ORGANOMETALLICS, 1999, Seiten 4325-4336, XP002143261**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von 1,2-Diimin-Verbindungen, Metallocenyl-substituierte 1,2-Diimin-Verbindungen, Kataly-satoren mit Metallocenyl-substituierten 1,2-Diimin-Liganden, Verfahren zu deren Herstellung sowie deren Einsatz in der Polymerisation ungesättigter Verbindungen.

Der Einsatz von Metallocen-Katalysatoren in der Polymerisation von ungesättig-ten Verbindungen zeigt einen großen Einfluß auf die Herstellung von Polyolefinen, da er einen Zugang zu neuartigen polyolefinischen Materialien oder zu Materialien mit verbesserten Eigenschaften eröffnet. Daher besteht großes In-teresse an der Entwicklung neuartiger Familien von Katalysatoren für die Poly-merisation ungesättigter Verbindungen, um eine noch bessere Kontrolle über die Eigenschaften von Polyölefinen oder weitere neuartige Produkte zu erhalten.

Insbesondere der Einsatz von Übergangsmetallkatalysatoren mit späten Übergangsmetallen (insbesondere Übergangsmetallen der VIII. Nebengruppe des Periodensystems der Elemente) ist, aufgrund deren Eigenschaft, Heteroatom-Funktionalitäten zu tolerieren, interessant. Nachteilig ist jedoch, daß die Übergangsmetallkatalysatoren mit späten Übergangsmetallen im Gegensatz zu den Übergangsmetallkatalysatoren mit frühen Übergangsmetallen (insbesondere Übergangsmetallen der III. bis V. Nebengruppe des Periodensystems der Elemente), aufgrund von konkurrierender β-Hydrideliminierung, häufig zu Dimerisierung bzw. Oligomerisierung von ungesättigten Verbindungen neigen.

Aus dem Stand der Technik sind Übergangsmetallkafcalysatoren später Über-gangsmetalle bekannt, die zur Polymerisation von ungesättigten Verbindungen geeignet sind.

In V. C. Gibson et al., Chem. Commun. 1998, 849-850 und M. Brookhart et al., J. Am. Chem. Soc. 1998, 120, 4049-4050, sind neue Olefin-Polymerisationskataly-satoren auf Basis von Fe(II) und Co(II) offenbart. Diese Katalysatoren tragen 2,6- Bis(imino)pyridyl-Liganden, die an den Iminostickstoffatomen arylsubstituiert sind, und zeigen hohe Aktivitäten in der Polymerisation von Ethlyen. Das erhaltene Polyethylen ist im wesentlichen linear und das Molekulargewicht ist stark abhängig von den Substituenten am Arylrest.

H. tom Dieck, Z. Naturforsch. 1981, 36b, 823-832, betrifft Bis(diazadien)nickel (0)-Komplexe mit aromatischen Substituenten am Stickstoffatom, sowie deren Konformationen in Abhängigkeit von den Substituenten am aromatischen Rest.

In M. Brookhart et al., J. Am. Chem. Soc. 1995, 117, 6415-6415 werden Kataly-satoren auf Basis von Pd(II) und Ni(II) für die Polymerisation von Ethylen und α-Olefinen beschrieben. Diese Katalysatoren tragen 1,2-Diimin-Liganden. Bei der Polymerisation von Ethylen und α-Olefinen werden damit Polymere mit hohem Molekulargewicht erhalten. In Abhängigkeit vom Ligandsystem, Metall, der Temperatur und dem Druck kann die Verzweigung von mit diesen Katalysatoren hergestelltem Polyethylen von stark bis wenig verzweigt eingestellt werden. Gemäß M. Brookhart et al., J. Am. Chem. Soc. 1996, 118, 267-268, ist mit diesen Katalysatoren mit Pd(II) als Metall auch die Copolymerisation von Ethylen und Propylen mit funktionalisierten Vinylmonomeren möglich.

WO 96/23010 betrifft Verfahren zur Polymerisation und Copolymerisation von Olefinen wie Ethylen, acrylischen Olefinen und anderen. Als Katalysatoren werden Übergangsmetallverbindungen mit Metallen aus der Gruppe Ti, Zr, Sc, V, Cr, Seltenerdmetallen, Se, Co, Ni und Pd eingesetzt. Als Ligandsysteme werden Diimin-Ligandsysteme, insbesondere 1,2-Diimin-Ligandsysteme, offenbart.

Aufgabe der vorliegenden Erfindung ist es, einen neuen Katalysator mit einem Übergangsmetall der VIII. Nebengruppe des Periodensystems der Elemente (spätes Übergangsmetall) als Zentralmetall für die Polymerisation ungesättigter Verbindungen bereitzustellen. Diese Aufgabe untergliedert sich in die Bereitstellung eines Ligandsystems für diesen Katalysator, sowie ein Verfahren zur Herstellung dieses Ligandsystems und die Bereitstellung eines Verfahrens zur Herstellung des entsprechenden Katalysators.

Diese Aufgabe wird gelöst durch ein Verfahren zur Herstellung von 1,2-Diimin-Verbindungen der allgemeinen Formel I,

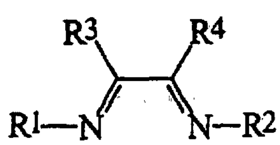

I

in der die Symbole die folgende Bedeutung haben

$R^1$, $R^2$ unabhängig voneinander Alkyl-, Aryl- oder Metallocenyl-Reste

und

$R^3$, $R^4$ unabhängig voneinander H, Alkyl- oder Arylreste,

oder

$R^3$ und $R^4$ bilden gemeinsam einen cyclischen Rest, so daß, einschließlich der beiden Imin-Kohlenstoffatome, ein 5-bis 8-gliedriger Ring gebildet wird, der gesättigt oder ungesättigt und unsubstituiert oder beliebig mit Kohlenwasserstoffresten substituiert sein kann,

durch Umsetzung von 1,2-Dicarbonylverbindungen mit primären Aminen.

Das erfindungsgemäße Verfahren ist dadurch gekennzeichnet, daß die Amine vor der Umsetzung mit den 1,2-Dicarbonylverbindungen mit Trialkylaluminium-Verbindungen aktiviert werden.

Das erfindungsgemäße Verfahren ist insbesondere geeignet zur Herstellung von 1,2-Diimin-Verbindungen mit voluminösen, sterisch anspruchsvollen Resten $R^1$ bis $R^4$, die ohne Zugabe von Trialkylaluminium-Verbindungen nicht erhalten werden können. Durch das erfindungsgemäße Verfahren sind somit neue Gruppen von 1,2-Diimin-Verbindungen zugänglich, die als Ligandsysteme für neuartige Katalysatoren eingesetzt werden können.

Unter Alkylresten sind für $R^1$, $R^2$, $R^3$ und $R^4$ lineare, verzweigte oder cyclische Alkylreste zu verstehen, bevorzugt $C_1$- bis $C_{20}$-Alkylreste, besonders bevorzugt $C_1$-bis $C_8$-Alkylreste.

Unter Arylresten sind unsubstituierte und substituierte Arylreste zu verstehen, bevorzugt $C_6$- bis $C_{20}$-Aryl, besonders bevorzugt substituierte $C_6$- bis $C_{14}$-Arylreste, die einfach oder mehrfach substituiert sein können, ganz besonders bevorzugt sind mit $C_1$bis $C_6$-Alkylresten substituierte $C_6$- bis $C_{10}$-Arylreste wie 4-Methylphenyl, 2,6-Dimethylphenyl, 2,6-Diethylphenyl, 2,6-Diisopropylphenyl, 2-tert.-Butylphenyl, 2,6-Di(tert.-butyl)phenyl oder 2-1-Propyl-6-methylphenyl.

Gemäß der vorliegenden Erfindung können $R^3$ und $R^4$ in der allgemeinen Formel I gemeinsam einen cyclischen Rest bilden, so daß, einschließlich der beiden Imin-Kohlenstoffatome, ein 5- bis 8-gliedriger Ring gebildet wird, der gesättigt oder ungesättigt und unsubstituiert oder beliebig mit Kohlenwasserstoffresten substituiert sein kann.

Unter Kohlenwasserstoffresten sind lineare, verzweigte oder cyclische Kohlenwasserstoffreste zu verstehen, die gesättigt oder ein- oder mehrfach ungesättigt sein können. Die cyclischen Kohlenwasserstoffreste können gemeinsam mit dem 5- bis 8-gliedrigen Ringsystem ein anelliertes System bilden, wobei das System ortho- oder ortho- und peri-anelliert sein kann. Bevorzugt weisen die Kohlenwasserstoffreste 1 bis 20 Kohlenstoffatome auf. Besonders bevorzugt ist eine ankondensierte Naphtho-Gruppe, die unsubstituiert oder ein- oder mehrfach mit Alkylresten oder Arylresten substituiert sein kann, so daß, einschließlich des aus $R^3$ und $R^4$ gemeinsam gebildeten 5- bis 8-gliedrigen Ringsystems, ein ortho- und perianelliertes System gebildet wird.

Enthalten in einem anellierten System (mindestens zwei Ringe mit mindestens je fünf Ringgliedern, worin die gemeinsamen Atome einbegriffen sind) zwei ringe zwei Atome gemeinsam oder enthält das System mehrere Ringe mit je zwei gemeinsamen Atomen, so ist das System ortho-anelliert. Die Zahl seiner gemeinsamen Atome ist dann doppelt so groß wie die Zahl der gemeinsamen Seiten. Hat ein Ring eines anellierten Systems zwei gemeinsame Atome mit jedem von zwei oder mehr Ringen einer Serie benachbarter Ringe, so ist das System ortho- und peri-anelliert und die Zahl der Atome beträgt weniger als das Doppelte der Zahl der gemeinsamen Seiten.

Unter Metallocenylresten sind Bis($\eta^5$-Cyclopentadienyl)metall-Reste zu verstehen, worin das Metall bevorzugt Fe, $Co^+$, Ni, Ru, Os, $Rh^+$ oder $Ir^+$ ist. Besonders bevorzugt ist das Metall Fe, d.h., es handelt sich um einen Bis ($\eta^5$-Cyclopentadienyl)eisen-Rest (Ferrocenylrest). Die Cyclopentadienylreste können substituiert oder unsubstituiert sein, wobei die Bindung des Rests an das Imin-Stickstoffatom in der allgemeinen Formel I über einen der beiden Cyclopentadienylreste erfolgt. Bevorzugte Substituenten am Cyclopentadienylrest sind -Me, $-SiMe_3$ oder $-SicHexMe_2$.

Bevorzugt werden in dem erfindungsgemäßen Verfahren Verbindungen der allgemeinen Formel I hergestellt, in denen die Reste $R^1$ und $R^2$ unabhängig voneinander substituierte Aryl- oder Metallocenylreste sind. Besonders bevorzugt sind $R^1$ und $R^2$ Ferrocenylreste. Ganz besonders bevorzugt sind Ferrocenylreste der allgemeinen Formeln IIa, IIb oder IIc,

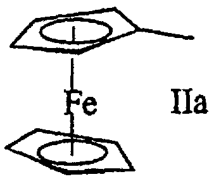
IIa
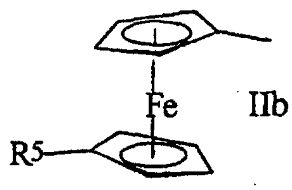
IIb
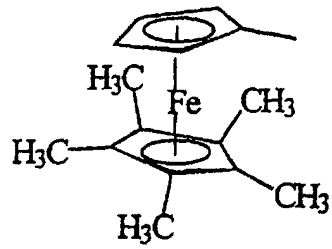
IIc

in denen $R^5$ -Me, $-SiMe_3$ oder $-SicHexMe_2$ ist.

In dem erfindungsgemäßen Verfahren werden primäre Amine der allgemeinen Formel III eingesetzt,

$$R^1—NH_2 \text{ oder } R^2—NH_2 \qquad \text{(III)},$$

in der $R^1$ und $R^2$ wie vorstehend definiert sind.

Die Herstellung der besonders bevorzugt eingesetzten Ferrocenylamine IIIa erfolgt, im Falle des unsubstituierten Ferrocenylamins, gemäß M. Herberhold in Organometallic Synthesis 3, R. E. King und J. J. Eisch (Editoren), Elsevier, Amsterdam (1986) 81-83. Dazu wird Ferrocencarbonsäure zunächst zu Ferrocenylazid umgesetzt, welches in einer Variante der Curtius-Umlagerung in Essigsäureanhydrid zu N-Acetylferrocenylamin (Monoferrocenylacetamid) umgesetzt wird. Durch Hydrolyse des N-Acetylferrocenylamins mit wäßrigem Kaliumhydroxid wird Ferrocenylamin erhalten.

Silylsubstituierte Ferrocenylamine IIIb können erhalten werden, indem man N-Acetylferrocenylamin mit einem Überschuß Chlorsilan, beispielsweise Trimethylchlorsilan oder Cyclohexyldimethylchlorsilan, umsetzt. Das erhaltene Acetamid kann wiederum durch Hydrolyse mit wäßrigem Kaliumhydroxid zu dem entsprechenden Amin umgesetzt werden. Fig. 1 zeigt ein Reaktionsschema zur Darstellung ausgewählter Ferrocenylamine.

Ferrocenylamine mit einem Pentamethylcyclopentadienylrest IIIc können erhalten werden, indem zunächst 1-, 2-, 3-, 4-, 5-Pentamethylferrocen-1-carbonsäure gemäß B. Bildstein et al., J. of Organomet. Chem. 540 (1997) 127-145 hergestellt wird. Dazu wird 1-, 2-, 3-, 4-, 5-Pentamethylferrocen zunächst mit n-Butylithium und Kalium-tert. -Butanolat und anschließend mit festem Kohlendioxid umgesetzt. Die erhaltene Carbonsäure wird gemäß Bildstein et al. mit Phosphorpentachlorid zu dem entsprechenden Säurechlorid umgesetzt. Nach Entfernung des Lösungsmittels und anderer flüchtiger Bestandteile wird der Rückstand in einem organischen Lösungsmittel, im allgemeinen in einem aromatischen Lösungsmittel, bevorzugt in Toluol, gelöst und, bevorzugt mit Natriumazid unter Phasentransferkatalyse, z.B. mit Benzyltriethylammoniumbromid als Phasentransferkatalysator, zu dem entsprechenden Carbonsäureazid umgesetzt. Nach wäßriger Aufarbeitung des Azids, die nach dem Fachmann bekannten Methoden erfolgt, und Reinigung, z.B. durch Chromatographie, wird das erhaltene Carbonsäureazid in Essigsäureanhydrid zu 1-, 2-, 3-, 4-, 5-Pentamethyl-ferrocen-1-yl-acetamid umgesetzt. Die Herstellung des entsprechenden Aminoferrocens erfolgt durch wäßrige Hydrolyse des Acetamids mit Kaliumhydroxid.

Statt einer wäßrigen Hydrolyse mit Kaliumhydroxid können die entsprechenden Ferrocenylacetamide auch in einem Alkohol, bevorzugt Ethanol, mit Kaliumhy-droxid zu den entsprechenden Aminoferrocenen hydrolysiert werden. Die Aufar-beitung der Aminoferrocene erfolgt in üblicher Weise, z.B. durch Extraktion mit Diethylether und anschließende Entfernung des Ethers im Vakuum. In Fig. 2 ist die Synthese von Ferrocenylaminen mit einem pentamethylsubstituierten Cyclopentadienylring exemplarisch dargestellt.

Als 1,2-Dicarbonylverbindungen werden in dem erfindungsgemäßen Verfahren Verbindungen der allgemeinen Formel IV eingesetzt:

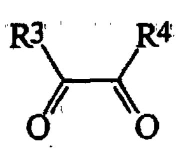
IV

in der $R^3$ und $R^4$ die vorstehende Bedeutung haben. Bevorzugt sind $R^3$ und $R^4$ unabhängig voneinander H oder $C_1$- bis $C_{20}$-Alkylreste, oder $R^3$ und $R^4$ bilden gemeinsam einen cyclischen Rest, der, einschließlich der beiden Carbonylkohlenstoffatome einen 5- bis 8-gliedrigen Ring bildet, der gesättigt oder ungesättigt und unsubstituiert oder beliebig mit Kohlenwasserstoffresten, wie vorstehend definiert, substituiert sein kann. Besonders bevorzugt werden Carbonylverbindungen der allgemeinen Formeln IVa, IVb oder IVc eingesetzt,

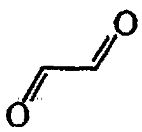 IVa 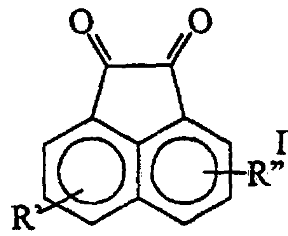 IVb 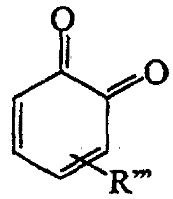 IVc

in denen R', R", R"' = H, Alkyl oder Aryl bedeuten.

In dem erfindungsgemäßen Verfahren werden die primären Amine vor ihrer Um-setzung mit den Dicarbonylverbindungen mit Trialkylaluminium-Verbindungen aktiviert. Dabei werden vorzugsweise Trialkylaluminium-Verbindungen der allgemeinen Formel V eingesetzt.

$$R^6R^7R^8Al \qquad V$$

Darin sind $R^6$, $R^7$, $R^8$ unabbängig voneinander $C_1$- bis $C_{10}$-Alkylreste. Besonders bevorzugt sind $R^6$, $R^7$ und $R^8$ unabhängig voneinander $C_1$- bis $C_3$-Alkylreste wie Methyl, Ethyl und i-Propyl. Ganz besonders bevorzugt sind $R^6$, $R^7$ und $R^8$ Methyl.

Durch Umsetzung eines primären Amins der allgemeinen Formel III mit Trialkylaluminium-Verbindungen der allgemeinen Formel V werden alktivierte Amine der allgemeinen Formel VI erhalten,

$$\left[ R^1—NH—AlR^6R^7 \right]_n \text{ oder } \left[ R^2—NH—AlR^6R^7 \right]_n \quad (VI)$$

in der die Reste $R^1$, $R^2$, $R^6$ und $R^7$ wie vorstehend definiert sind und n = 2 bis 4 ist. Bei der besonders bevorzugten Herstellung von ferrocenylsubstituierten 1,2-Diimin-Verbindungen werden, durch die bevorzugte Umsetzung des entsprechenden Ferrocenylamins mit Trimethylaluminium, aktivierte Amine der allgemeinen Formel VIa erhalten,

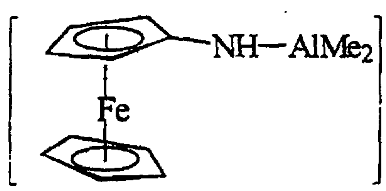 (VIa)

in der n = 2 bis 4 ist.

Das erfindungsgemäße Verfahren erfolgt im allgemeinen in zwei Schritten:

A Herstellung des aktivierten Amins;

B Umsetzung des aktivierten Amins mit einer 1,2-Dicarbonylverbindung der allgemeinen Formel IV.

A Herstellung des aktivierten Amins:

Ein primäres Amin der allgemeinen Formel III wird mit einer Trialkylaluminium-Verbindung in einem Molverhältnis von im allgemeinen 1,5:1 bis 1:1,5, bevorzugt von 1,3:1 bis 1:1,3, ganz besonders bevorzugt von etwa 1:1 in einem Lösungsmittel zusammengegeben. Als Lösungsmittel sind organische Lösungs-mittel geeignet, die vorzugsweise wasserfrei sind, besonders bevorzugt sind wasserfreie aromatische Lösungsmittel, von denen Toluol ganz besonders bevorzugt ist. Die Reaktionsmischung wird bei einer Temperatur, die von den eingesetzten Reaktionspartnern und vom Lösungsmittel abhängig ist und im allgemeinen zwischen Raumtemperatur und 110°C, bevorzugt zwischen 50 und 80°C, besonders bevorzugt bei etwa 70°C liegt, gerührt. Nach einer Reaktionszeit, die im allgemeinen von den Reaktionspartnern abhängig ist und üblicherweise 10 Minuten bis 5 Stunden, bevorzugt 1 bis 4 Stunden, besonders bevorzugt etwa 3 Stunden beträgt, erfolgt in situ, ohne weitere Aufarbeitung, die zweite Stufe des erfindungsgemäßen Verfahrens.

B Umsetzung des aktivierten Amins mit einer 1,2-Dicarbonylverbindung:

Zu der gemäß A erhaltenen Reaktionsmischung wird eine 1,2-Dicarbonylver-bindung der allgemeinen Formel IV zugegeben. Das molare Verhältnis zwischen der 1,2-Dicarbonylverbindung und dem eingesetzten primären Amin beträgt im allgemeinen 1:6 bis 1:2, bevorzugt 1:4 bis 1:2. Die Reaktionstemperatur beträgt im allgemeinen, in Abhängigkeit vom Lösungsmittel und den Reaktionspartnern, Raumtemperatur bis 110°C, bevorzugt 50 bis 110°C, besonders bevorzugt 70 bis 110°C, und die Reaktionszeit liegt üblicherweise zwischen 30 Minuten und 2 Tagen, bevorzugt zwischen 5 Stunden und 24 Stunden, besonders bevorzugt zwischen 8 und 16 Stunden. Die Aufarbeitung erfolgt in üblicher Weise, z.B. durch Entfernen des Lösungsmittels im Vakuum und anschließende Reinigung des erhaltenen Produktes mittels Chromatographie. Die Fig. 3 und 4 zeigen jeweils exemplarisch die Herstellung von ferrocenylsubstituierten 1,2-Diimin-Verbindungen.

Das erfindungsgemäße Verfahren ermöglicht die Herstellung von 1,2-Diimin-Ver-bindungen mit sterisch anspruchsvollen Resten, die auf andere Weise nur schwer oder gar nicht zugänglich sind. Ein weiterer Gegenstand der vorliegenden Erfindung ist daher die Verwendung von Trialkylaluminium-Verbindungen zur Herstellung von 1,2-Diimin-Verbindungen aus 1,2-Dicarbonylverbindungen und primären Aminen.

Durch das erfindungsgemäße Verfahren bzw. die erfindungsgemäße Verwendung sind neuartige 1,2-Diimin-Verbindungen zugänglich, die an den Imin-Stickstoff-atomen Metallocenylreste aufweisen. Ein weiterer Gegenstand der vorliegenden Erfindung sind daher Verbindungen der allgemeinen Formel I,

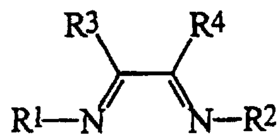
I

in der die Symbole die folgende Bedeutung haben,

$R^3$, $R^4$ unabhängig voneinander H, Alkyl- oder Arylreste bedeuten,

oder

$R^3$ und $R^4$ bilden gemeinsam einen cyclischen Rest, so daß, einschließlich der beiden Imin-Kohlenstoffatome ein 5- bis 8-gliedriger Ring gebildet wird, der gesättigt oder ungesättigt und unsubstituiert oder beliebig mit Kohlenwasserstoffresten substituiert sein kann,

und in der $R^1$ und $R^2$ unabhängig voneinander Metallocenylreste sind.

Die erfindungsgemäßen Verbindungen werden vorzugsweise nach dem erfindungsgemäßen Verfahren hergestellt. Bevorzugt sind in den Verbindungen der allgemeinen Formel I $R^3$ und $R^4$ = H oder $C_1$- bis $C_{20}$-Alkyl oder $R^3$ und $R^4$ bilden gemeinsam einen cyclischen Rest, so daß, einschließlich der beiden Imin-Kohlenstoffatome ein 5-bis 6-gliedriger Ring gebildet wird, der gesättigt oder ungesättigt und unsubstituiert oder beliebig mit Kohlenwasserstoffresten substituiert sein kann. Die Metallocenyl-Reste enthalten bevorzugt ein Metall aus der Gruppe Fe, $Co^+$, Ni, Ru, Os, $Rh^+$ und $Ir^+$. Besonders bevorzugt wird Fe (Ferrocenylreste) eingesetzt.

Die Cyclopentadienylreste können substituiert oder unsubstituiert sein, wobei die Bindung zum Imin-Stickstoffatom in der allgemeinen Formel I über einen der beiden Cyclopentadienylreste erfolgt. Bevorzugte Substituenten sind -Me, $-SiMe_3$ oder $-SicHexMe_2$.

Besonders bevorzugt sind Verbindungen der allgemeinen Formeln Ia, Ib und Ic.

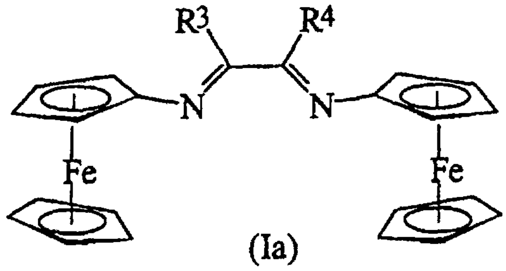

(Ia)

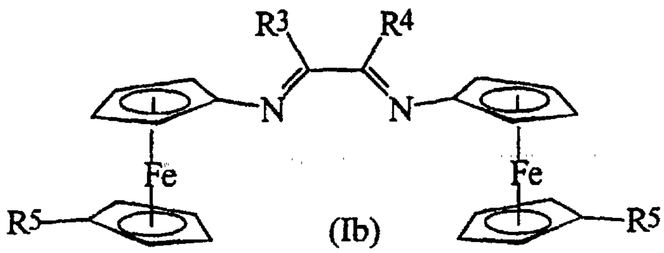

(Ib)

worin $R^5$ = $-SiMe_3$ ($Ib_1$) oder $-SicHexMe_2$ ($Ib_2$) ist;

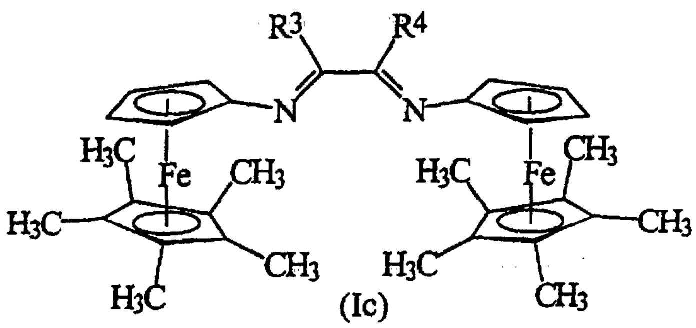

(Ic)

Die erfindungsgemäßen Verbindungen sind als Liganden für Katalysatoren, die zur Polymerisation ungesättigter Verbindungen eingesetzt werden können, geeignet. Insbesondere sind die erfindungsgemäßen Verbindungen als Liganden für Katalysatoren mit einem Metall der späten Übergangsmetalle, d.h. mit einem Metall der VIII. Nebengruppe des Periodensystems der Elemente geeignet. Ein weiterer Gegenstand der vorliegenden Erfindung sind daher Verbindungen der allgemeinen Formel VII.

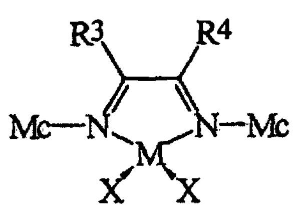

(VII),

in der

$R^3$ und $R^4$ unabhängig voneinander H, Alkyl- oder Arylreste bedeuten,

oder

$R^3$ und $R^4$ gemeinsam einen cyclischen Rest bilden, so daß, einschließlich der beiden Imin-Kohlenstoffatome, ein 5- bis 8-gliedriger Ring gebildet wird, der gesättigt oder ungesättigt und unsubstituiert oder beliebig mit Kohlenwasserstoffresten substituiert sein kann;

Metallocenyl (Mc) ein am Cyclopentadienylrest unsubstituierter oder substituierter Metallocenylrest,

M ein Übergangsmetall der VIII. Nebengruppe des Periodensystems der Elemente, und

X Halogenid oder ein $C_1$- bis $C_{20}$-Alkylrest

sind.

Die bevorzugten Reste $R^3$, $R^4$ und Metallocenyl (Mc) wurden vorstehend beschrieben.

Das Übergangsmetal M der VIII. Nebengruppe des Periodensystems der Elemente ist bevorzugt Pd, Co, Ni oder Fe. Besonders bevorzugt sind Pd und Ni. Die Liganden X können unabhängig voneinander Halogenid oder Alkylreste sein. Bevorzugt handelt es sich um Chlorid-, Bromid- oder Methylreste. Besonders bevorzugt ist als Gruppe $MX_2$: $PdCl_2$, $Pd(Cl)CH_3$, $NiCl_2$, $CoCl_2$, $NiBr_2$ oder $FeCl_2$.

Ganz besonders bevorzugt sind Verbindungen der allgemeinen Formeln VIIa, VIIb und VIIc.

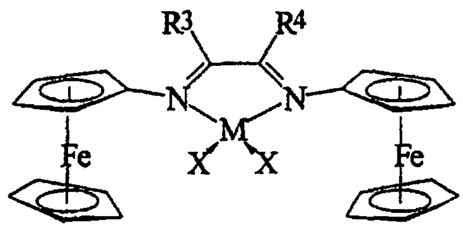

(VIIa)

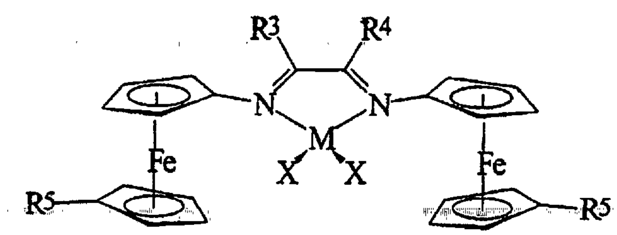

(VIIb)

worin $R^5$ = $-SiMe_3$ ($VIIb_1$) oder -SicHexMe$_2$ ($VIIb_2$) ist;

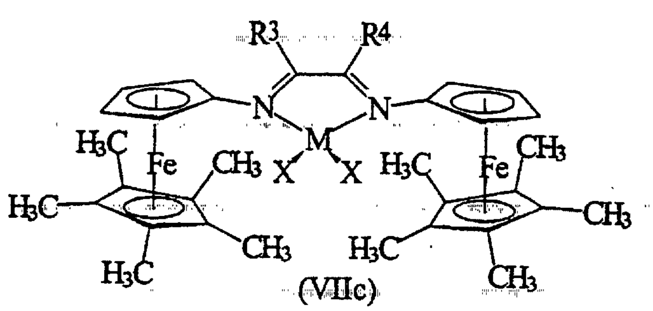

(VIIc)

Die Herstellung der erfindungsgemäßen Verbindungen der allgemeinen Formel VII erfolgt üblicherweise durch Umsetzung der entsprechenden Verbindungen der allgemeinen Formel I mit Übergangsmetallsalzen von Metallen der VIII. Nebengruppe des Periodensystems der Elemente.

In einer bevorzugten Ausführungsform wird eine als Ligand geeignete Verbindung der allgemeinen Formel I

in einem organischen Lösungsmittel, z.B. Tetrahydrofuran (THF) oder Methylenchlorid, mit einem entsprechenden Metallsalz, z.B. $NiCl_2$(DME) (DME = 1,2-Dimethoxyethan), $NiBr_2(DME)_2$, $CoCl_2$, $PdCl_2$ (Benzonitril)$_2$, PdClMe(COD) (COD = 1,5-Cyclooctadien) zusammengegeben. Das Molverhältnis von Ligand zu Metallsalz beträgt im allgemeinen 1,5:1 bis 1:1,5, bevorzugt 1,2:1 bis 1:1,2, besonders bevorzugt ca. 1:1. Die Reaktionsmischung wird bei Temperaturen von im allgemeinen Raumtemperatur bis 50°C, bevorzugt von Raumtemperatur bis 40°C, besonders bevorzugt bei Raumtemperatur für im allgemeinen 0,5 Stunden bis 16 Stunden, bevorzugt 1 bis 6 Stunden, besonders bevorzugt 1 bis 3 Stunden gerührt. Die Aufarbeitung erfolgt in üblicher Weise, z.B. durch Entfernung des Lö-sungsmittels im Vakuum, Waschen des Rückstandes mit einem Lösungsmittel, in dem der Rückstand (Produkt) weitgehend unlöslich ist, z.B. mit Diethylether, gegebenenfalls Digerieren in einem unpolaren Lösungsmittel, z.B. Hexan, Abfiltrieren, Waschen und Trocknen.

In Fig. 5 ist die Herstellung der erfindungsgemäßen Metallkomplexe exemplarisch dargestellt. Darin bedeuten:

**VII** Metallkomplex
**I** Diazabutadien
**$MX_2$** Metallsalz

Die erfindungsgemäßen Metallkomplexe der allgemeinen Formel VII sind als Katalysatoren für die Polymerisation ungesättigter Verbindungen geeignet. Ein weiterer Gegenstand der vorliegenden Erfindung ist daher die Verwendung von Verbindungen der allgemeinen Formel VII als Katalysatoren in einem Verfahren zur Polymerisation ungesättigter Verbindungen, sowie ein Verfahren zur Herstellung von Polyolefinen durch Polymerisation ungesättigter Verbindungen in Anwesenheit des erfindungsgemäßen Katalysators und eines Aktivators.

Es ist bekannt, daß die Strukturen von Polymeren und somit auch deren Eigen-schaften und Verwendungszweck von dem in der Polymerisation eingesetzten Katalysator sowie den Reaktionsbedingungen während der Polymerisation abhängen. Die erfindungsgemäßen Katalysatoren stellen somit eine Möglichkeit dar, neuartige Polymere mit spezifischen Eigenschaftsprofilen bereitzustellen.

Als Aktivatoren (Cokatalysatoren) sind insbesondere starke, neutrale Lewissäuren, ionische Verbindungen mit lewissauren Kationen und ionische Verbindungen mit Brönsted-Säuren als Kationen geeignet.

Als starke, neutrale Lewissäuren sind Verbindungen der allgemeinen Formel VIII bevorzugt,

$$\mathbf{M'X^1X^2X^3} \qquad \mathbf{(VIII)}$$

in der die Symbole die folgende Bedeutung haben

M' ein Element der III. Hauptgruppe des Periodensystems der Elemente, bevorzugt B, Al oder Ga, besonders bevorzugt B,

$X^1$, $X^2$, $X^3$ unabhängig voneinander Wasserstoff, $C_1$- bis $C_{10}$-Alkyl, $C_6$- bis $C_{15}$-Aryl, Alkylaryl, Arylalkyl, Halogenalkyl oder Halogenaryl mit jeweils 1 bis 10 Kohlenstoffatomen im Alkylrest und 6 bis 20 Kohlenstoffatomen im Arylrest oder Fluor, Chlor, Brom oder Iod stehen, bevorzugt für Halogenaryle, besonders bevorzugt für Pentafluorphenyl.

Ganz besonders bevorzugt sind Verbindungen der allgemeinen Formel VIII, in der $X^1$, $X^2$, $X^3$ gleich sind, bevorzugt Tris(pentafluorphenyl)boran.

Geeignete ionische Verbindungen mit lewissauren Kationen sind Verbindungen der allgemeinen Formel IX,

$$[(Y^{a+})Q_1Q_2...Q_z]^{d+} \qquad (IX)$$

in der die Symbole die folgenden Bedeutungen haben

Y ein Element der I. bis VI. Haupcgruppe oder der I. bis VIII. Nebengruppe des Periodensystems der Elemente,

$Q_1$ bis $Q_z$ einfach negativ geladene Reste wie $C_1$- bis $C_{28}$-Alkyl, $C_6$- bis $C_{15}$-Aryl, Alkylaryl, Arylalkyl, Halogenalkyl, Halogenaryl mit jeweils 6 bis 20 Kohlenstoffatomen im Aryl- und 1 bis 28 Kohlenstoffatomen im Alkylrest. $C_1$- bis $C_{10}$-Cycloalkyl, welches gegebenenfalls mit $C_1$- bis $C_{10}$-Alkylgruppen substituiert sein kann, Ha-

logenid, $C_1$- bis $C_{28}$-Alkoxy, $C_6$- bis $C_{15}$-Aryloxy, Silyl- oder Mercaptylgruppen,

a ganze Zahlen von 1 bis 6,

z ganze Zahlen von 0 bis 5,

d Differenz von a-z, wobei d jedoch größer oder gleich 1 ist,

Besonders geeignet sind Carboniumkationen, Oxoniumkationen und Sulfoniumkationen sowie kationische Übergangsmetallkomplexe. Insbesondere sind das Triphenylmethylkation, das Silberkation und das 1,1'-Dimethylferrocenylkation zu nennen. Bevorzugt besitzen sie nicht koordinierende Gegenionen, insbesondere Borverbindungen, wie sie auch in WO 91/09882 genannt werden, bevorzugt Tetrakis (pentafluorophenyl)borat.

Ionische Verbindungen mir Brönsted-Säuren als Kation und vorzugsweise ebenfalls nicht koordinierenden Gegenionen sind in WO 91/09882 genannt, bevorzugtes Kation ist N,N-Dimethylanilinium.

In einer besonderen Ausführungsart des erfindungsgemässen Verfahrens werden Methylaluminoxan oder N, N-Dimethylanilinium-tetrakis(pentafluorophenyl)borat als Aktivator eingesetzt.

Die Menge an Aktivator beträgt bevorzugt 0,1 bis 10 Äquivalente, bezogen auf den Katalysator VII.

Das erfindungsgemäße Polymerisationsverfahren ist zur Herstellung von Homo- oder Copolymeren geeignet. Bevorzugt eingesetzte ungesättigte Verbindungen bzw. Kombinationen ungesättigter Verbindungen sind dabei ungesättigte Verbindungen ausgewählt aus Ethylen, $C_3$- bis $C_{20}$-Monoolefinen, Ethylen und $C_3$- bis $C_{20}$-Monoolefinen, Cycloolefinen, Cycloolefinen und Ethylen und Cycloolefinen und Propylen. Bevorzugte Cycloolefine sind Norbonen, Norbonadien und Cyclopenten.

Bei Einsatz des erfindungsgemäßen Katalysators mit Pd(II) als Zentralmetall M können die vorstehend genannten Monomere mit Monomeren, die eine Carbonylgruppe aufweisen, wie Estern, Carbonsäuren, Kohlenmonoxid und Vinylketonen copolymerisiert werden. Dabei sind die folgenden Kombinationen von ungesättigten Verbindungen bevorzugt: Ethylen, $C_3$- bis $C_{20}$-Monoolefine, Ethylen und $C_3$- bis $C_{20}$-Monoolefine, Ethylen und ein Alkylacrylat, insbesondere Methylacrylat, Ethylen und eine Acrylsäure, Ethylen und Kohlenmonoxid, Ethylen, Kohlenmonoxid und ein Acrylatester oder eine Acrylsäure, insbesondere Methylacrylat sowie Propylen und Alkylacrylat, insbesondere Methylacrylat.

Besonders bevorzugt wird nach dem erfindungsgemäßen Verfahren ein Polyethylenhomopolymer hergestellt, d.h. als ungesättigte Verbindung wird besonders bevorzugt Ethylen eingesetzt.

Je nach Reaktionsbedingungen und eingesetzten Monomeren ist es möglich, mit dem erfindungsgemäßen Verfahren Homopolymere, statistische Copolymere oder Blockcopolymere zu erhalten.

Die Polymerisation wird unter allgemein üblichen Bedingungen in Lösung, Suspension oder in der Gasphase durchgeführt. Dabei ist eine Polymerisation in Lösung bevorzugt.

Die erfindungsgemäßen Katalysatorsysteme können in Form von Vollkatalysatoren oder Trägerkatalysatoren, in Abhängigkeit von den Polymerisationsbedingungen, eingesetzt werden.

Als Trägermaterialien werden bevorzugt feinteilige Feststoffe eingesetzt, deren Teilchendurchmesser im Bereich von im allgemeinen 1 bis 200 $\mu$m liegen, bevorzugt von 30 bis 70 $\mu$m.

Geeignete Trägermaterialien sind beispielsweise Kieselgele, bevorzugt solche der Formel $SiO_2 \cdot a\ Al_2O_3$, worin a für eine Zahl im Bereich von 0 bis 2 steht, bevorzugt von 0 bis 0,5; es handelt sich also um Alumosilikate oder Siliciumdioxid. Derartige Produkte sind im Handel erhältlich, beispielsweise Silica Gel 332 von Grace oder ES 70x von Crosfield.

Diese Trägermaterialien können zur Entfernung von adsorbiertem wasser einer thermischen oder chemischen Behandlung unterzogen werden oder calciniert werden, wobei bevorzugt eine Behandlung bei 80 bis 200°C, besonders bevorzugt bei 100 bis 150°C, durchgeführt wird.

Andere anorganische Verbindungen wie $Al_2O_3$ oder $MgCl_2$ oder Mischungen, die diese Verbindungen enthalten, können ebenfalls als Trägermaterialien eingesetzt werden.

Als Lösungsmittel sind insbesondere aprotische organische Lösungsmittel geeignet. Dabei können das Katalysatorsystem, die oder das Monomere und das Polymer in diesen Lösungsmitteln löslich oder unlöslich sein, die Lösungsmittel sollten jedoch nicht an der Polymerisation teilnehmen. Geeignete Lösungsmittel sind Alkane, Cycloalkane, ausgewählte halogenierte Kohlenwasserstoffe und aromatische Kohlenwasserstoffe. Bevorzugte Lösungsmittel sind Hexan, Toluol und Benzol, besonders bevorzugt ist Toluol.

Die Polymerisationstemperaturen bei der Lösungspolymerisation liegen im allgemeinen in Bereichen von -20 bis 350°C, bevorzugt von 0 bis 100°C, besonders bevorzugt von Raumtemperatur bis 80°C. Der Reaktionsdruck beträgt im allgemeinen 0,1 bis 3000 bar, bevorzugt 1 bis 200 bar, besonders bevorzugt 1 bis 40 bar. Die Polymerisation kann in jeder für die Polymerisation ungesättigter Verbindungen geeigneten Apparatur durchgeführt werden.

Das erfindungsgemäße Polymerisationsverfahren eröffnet einen Zugang zu Polyolefinen mit neuartigen

Strukturen und Eigenschaften. Ein weiterer Gegenstand der vorliegenden Erfindung sind daher Polymere, herstellbar nach dem erfindungsgemäßen Verfahren.

Die nachfolgenden Beispiele erläutern die Erfindung zusätzlich.

**Beispiele**

1. Bezugsliste der hergestellten Verbindungen

1,2-Diiminliganden:

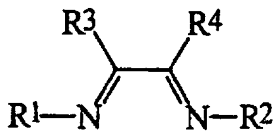

I

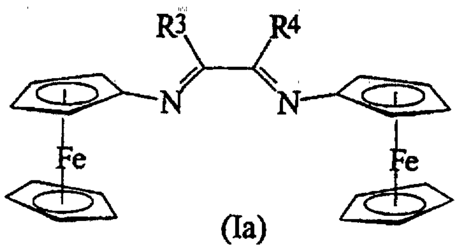

(Ia)

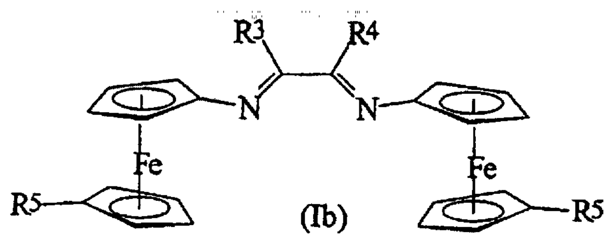

(Ib)

Ib1: R5 = —SiMe3

Ib2: R5 = —SiMe2cHex

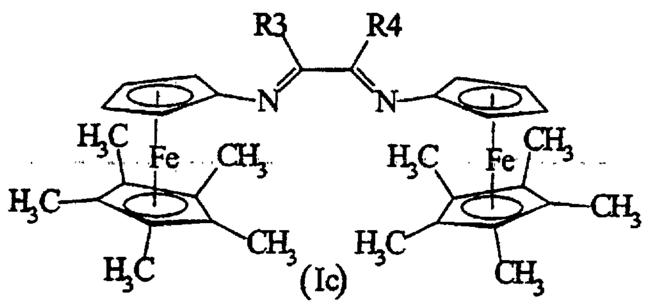

(Ic)

Kennzeichnung der Liganden mit unterschiedlichen Resten $R^3$ **und $R^4$:**

Ia', Ib1', $Ib_2$', Ic': $R^3$ und $R^4$ jeweils = H

Ia", Ib1", $Ib_2$", Ic":$R^3$ und $R^4$ zusammen: =

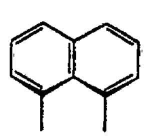

Amine

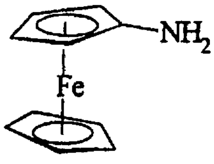

(IIIa)

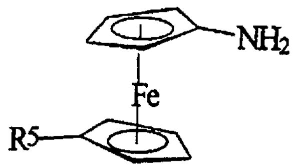

(IIIb)

$IIIb_1$: R5 = —$SiMe_3$

$IIIb_2$: R5 = —$SiMe_2$cHex

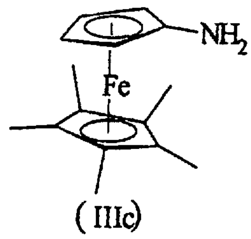

(IIIc)

Metallkomplexe:

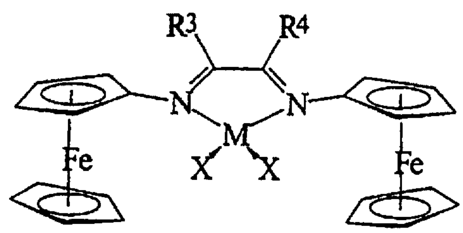

(VIIa)

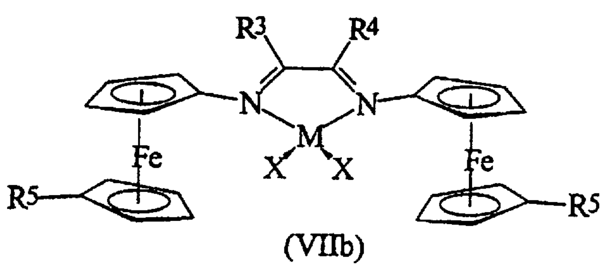

(VIIb)

$VIIb_1$: R5 = —$SiMe_3$

$VIIb_2$: R5 = —$SiMe_2$cHex

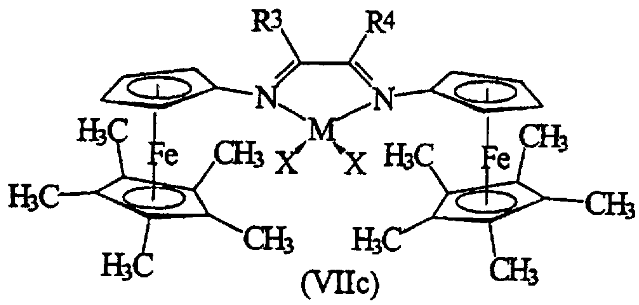

(VIIc)

Kennzeichnung der Metallkomplexe mit unterschiedlichen Resten $R^3$ **und $R^4$:**
VIIa', VIIb1', $VIIb_2$', VIIc': $R^3$ und $R^4$ jeweils = H
VIIa", VIIb1", $VIIb_2$", VIIc":$R^3$ und $R^4$ zusammen: =

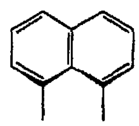

Kennzeichnung der Metallkomplexe mit unterschiedlichen Resten R3 und $R^4$ und unterschliedlichen Metallen am Beispiel des Komplexes $VIIb_1$':

| $MX_2$ | PdCl2 | Pd(Cl)CH3 | NiCl2 | CoCl2 | NiBr2 |
|---|---|---|---|---|---|
| Metallkomplex[1)] | $VIIb1'_a$ | $VIIb1'_b$ | VIIa'c[2)] | $VIIb1'_d$ | $VIIb1'_e$ |

1) Numerierung der Komplexe VII am Beispiel des Komplexes $VIIb_1$' ;
2) Numerierung der Komplexe VII am Beispiel des Komplexes VIIa', da dieser Komplex des Liganden $VIIb_1$' nicht hergestellt wurde.

2. Ligand- und Komplexsynthesen:

Die Ligand- und Komplexsynthesen wurden unter Ausschluß von Luft und Feuchtigkeit durchgeführt. Die verwendeten Apparaturen und Reagenzien wurden entsprechend vorbereitet.

Ligandsynthese

1-Acetamido-1'-substituierte Ferrocene:

N-(1'-Trimethylsilylferrocenyl)trimethylsilyacetamid:
Zu einer Lösung von 0,5g (2,1mmol) Monoferrocenylacetamid werden in 40 ml THF bei -80°C 4 ml (6 mmol) t-Butyllithium zugetropft. Anschließend wird langsam auf Raumtemperatur erwärmt. Nach einer Stunde Rühren bei Raumtemperatur wird ein Überschuß Trimethylchlorsilan zugegeben [1,3 ml (10 mmol) Trimethylchlorsilan]. Der Ansatz wird auf 100 ml Wasser gegossen und mehrmals mit Ether extrahiert und die vereinigten Etherphasen mit Wasser gewaschen. Die Reinigung erfolgt mittels Säulenchromatographie (Laufmittel $CH_2Cl_2$, $Al_2O_3$).

Ausbeute:

N-(1'-Trimethylsilylferrocenyl)trimethylsilyacetamid: 621mg (1,6 mmol, 76% d. Theorie)

Daten für N- (1'-Trimethylsilylferrocenyl)trimethylsilyacetamid: gelbe Kristalle, m.p.: 145°C, $C_{18}H_{29}FeNOSi_2$, $^1$H-NMR($CDCl_3$): δ 0.15 (s, 9H, $SiMe_3$), 0.20 (s, 9H, $SiMe_3$), 1.82 (s, 3H, $CH_3$), 3.91(m, 2H, $cp_{subst}$) (cp = Cyclopentadienyl), 4.05 (m, 2H, $cp_{subst}$), 4.30 (m, 2H, $cp_{subst}$), 4.49 (m, 2H, $cp_{subst}$).

N-[1'-(Dimethylcyclohexyl)silylferrocenyl]acetamid:

Zu einer Lösung von 2g (8,4mmol) Monoferrocenylacetamid werden in 40 ml THF bei -80°C 15,5 ml (25,2 mmol) t-Butyllithium zugetropft. Anschließend wird langsam auf Raumtemperatur erwärmt. Nach einer Stunde Rühren bei Raumtemperatur wird ein Überschuß Cyclohexyldimethylchlorsilan zugegeben [5 ml (23 mmol) Cyclohexyldimethylchlorsilan]. Der Ansatz wird auf 100 ml Wasser gegossen und mehrmals mit Ether extrahiert und die vereinigten Etherphasen mit Wasser gewaschen. Die Reinigung erfolgt mittels Säulenchromatographie (Laufmittel $CH_2Cl_2$, $Al_2O_3$).

Ausbeute:

N-[1'-(Dimethylcyclohexyl)silylferrocenyl]acetamid: 1,394g (2,7 mmol, 32% d. Theorie)

Daten für N-[1'-(Dimethylcyclohexyl)silylferrocenyl]acetamid: gelbes Öl, $C_{28}H_{43}FeNOSi_2$, $^1$H-NMR($CDCl_3$): δ 0.05 (s, 6H, $SiMe_2$), 0.50-2.00(m, 11H, cHex), 2.00 (s, 3H, $CH_3$), 3.94(m, 2H, $cp_{subst}$), 4.03 (m, 2H, $cp_{subst}$), 4.30 (m, 2H, $cp_{subst}$), 4.53 (m, 2H, $cp_{subst}$), 6.80 (s. 1H, N-H).

1', 2', 3', 4', 5'-Pentamethylferrocen-1-carbonsäureazid:

0,97g (3,24 mmol) 1', 2', 3', 4', 5'-Pentamethylferrocen-1-carbonsäure werden mit 0,683g (3,28 mmol) Phosphorpentachlorid in 30 ml Toluol in das Säurechlorid überführt, gemäß B. Bildstein, A. Hradsky, H. Kopacka, R. Malleier, K.H. Ongania, *J. of Organomet. Chem.* 540 (1997) 127. Nach sechs Stunden Rühren werden im Hochvakuum Toluol, Phosphor-oxitrichlorid und die anderen flüchtigen Bestandteile abgezogen. Der resultierende Rückstand wird in 30 ml absolutem Toluol gelöst und mit 0,21g (3,24 mmol) Natriumazid unter Phasen-transferkatalyse mit 0,737g (3,24 mmol) Benzyltriethylammoniumbromid zum 1', 2', 3', 4', 5'-Pentamethylferrocen-1-carbonsäureazid umgesetzt. Nach Rühren über Nacht wird wäßrig aufgearbeitet und chromatographiert ($Al_2O_3$, Laufmittel: Petrolether/Ether 1:1). Neben 0,13g 1', 2', 3', 4',5'-Pentamethylferrocen-1-carbonsäureazid werden 0,2g 1', 2', 3', 4', 5'-Pentamethylferrocen-1-carbonsäure zurückgewonnen, entsprechend einem Umsatz von 0,77g (2,56 mmol) Carbonsäure zu 0,13g Säureazid in 15,6% Ausbeute.

Daten für 1', 2', 3', 4', 5'-Pentamethylferrocen-l-carbonsäureazid: rote Kristalle, $C_{16}H_{19}FeN_3O$, $^1$H-NMR ($CDCl_3$): δ 1.23 (s, 15H, cp*) (cp* = 1', 2', 3', 4', 5'-Pentamethylcyclopentadienyl), 4.53(m, 2H, cp) (cp = Cyclopentadienyl), 4.87 (m, 2H, cp).

1', 2', 3', 4', 5'-Pentamethylferrocen-1-yl-acetamid:

0,13g (0,4mmol) 1', 2', 3', 4', 5'-Pentamethylferrocen-1-carbonsäureazid werden in 10 ml Essigsäureanhydrid mit 0,5 ml Essigsäure fünf Stunden bei 80°C gerührt. Nach wäßriger Aufarbeitung werden 0,102g 1', 2', 3', 4', 5'-Pentamethylferrocen-1-yl-acetamid erhalten.
Ausbeute: 0,102g (0,33 mmol, 82,5% d.Theorie)
Daten für 1', 2', 3', 4', 5'-Pentamethylferrocen-1-yl-acetamid: orange Kristalle, m.p.: 149°C, $C_{17}H_{23}FeNO$, $^1$H-NMR ($CDCl_3$): δ 1.86 (s, 15H, cp*), 1.97(s, 3H, $CH_3$), 3.63 (m, 2H, cp), 4.13(m, 2H, cp).

Aminoferrocene, IIIa, $IIIb_1$, $IIIb_2$, IIIc:

Aminoferrocen:

Aminoferrocen IIIa wurde gemäß M. Herberhold, M. Ellinger, L. Haumeier, *in Organometallic Synthesis 3*, R. B. King und J.J. Eisch (Editoren), Elsevier, Amsterdam (1986) 81 und G.R. Knox, P.L. Pauson, D. Willison, E. Solcanioca, S. Toma, *Organometallics 9* (1990) 301 (und darin zitierte Literatur) dargestellt.

Aminoferrocene $IIIb_1$, $IIIb_2$, IIIc, allgemeine Arbeitsvorschrift:

Das entsprechende Acetamid wird in eine Lösung von Kaliumhydroxid in 50 ml Ethanol gegeben und anschließend über Nacht unter Rückfluß erhitzt. Das Produkt wird auf Wasser gegossen, mehrmals mit Ether extrahiert und die vereinigten Etherphasen mit Wasser gewaschen, getrocknet und am Rotavapor einrotiert. Tabelle 1 zeigt die den Aminoferrocenen $IIIb_1$, $IIIb_2$, IIIc entsprechenden Daten.

Tabelle 1:

| Aminoferrocen | Acetamid | Menge Acetamid | Menge KOH | Ausbeute |
|---|---|---|---|---|
| $IIIb_1$ | N-(1'-Trimethylsilylferrocenyl) trimethylsilyacetamid | 0,45g (1,2 mmol) | 2,5g | 0,315g, 99% |
| $IIIb_2$ | N-(1'-[Dimethylcyclohexyl)silylferrocenyl] acetamid | 1,394g (2,7 mmol) | 7g | 0,827g, 91% |
| IIIc | 1',2',3',4',5'-Pentamethylferrocen-1-yl-acetamid | 0,102g (0,33 mmol) | 0,3g | 87mg, 97% |

Daten für **$IIIb_1$** (1-Amino-1'-trimethylsilylferrocen): gelbes Ö1, $C_{13}H_{19}FeNSi$, $^1H$-NMR($CDCl_3$): δ 0.22 (s, 9H, $SiMe_3$), 2.54(s, 2H, N-H), 3.77 (m, 2H, $cp_{subst}$), 3.92 (m, 2H, $cp_{subst}$), 3.98 (m, 2H, $cp_{subst}$), 4.24 (m, 2H, $cp_{subst}$).

Daten für **$IIIb_2$** (1-Amino-1'-dimethylcyclohexylferrocen): gelbes Öl, $C_{18}H_{27}FeNSi$, $^1H$-NMR($CDCl_3$): δ 0.22 (s, 9H, $SiMe_3$), 0.5-2.0 (m, 11H, cHex), 2.37 (s, 2H, N-H), 3.75 (m, 2H, $cp_{subst}$), 3.91 (m, 4H, 2*$cp_{subst}$), 4.21 (m, 2H, $cp_{subst}$).

Daten für **IIIc** (1-Amino-1',2',3',4',5'-pentamethylferrocen): braungelbes instabiles Öl, $C_{15}H_{21}FeN$, IR(KBr): ($cm^{-1}$) 3411m, 2964m, 2927m, 2875m, 1701w, 1638w, 1618w, 1493w, 1452w, 1379m, 1261m, 1094m, 1032m, 866w, 802s, 480w. MS (EI, 70 eV): m/z(%) 271(100) ($M^+$), 133.5(100) (cp*), 121(16) ($Fecp^+$).

1,4-Diferrocenyldiazabutadiene Ia', $Ib_1$', $Ib_2$', Ic':

Das entsprechende Ferrocenylamin wird in 40 ml Aceton und 20 ml Wasser gelöst und anschließend mit einem Überschuß 40%iger wäßriger Glyoxallösung versetzt. Nach dem Rühren über Nacht bei Raumtemperatur wird das violette Produkt mit 200 ml Wasser vollständig ausgefällt und anschließend abfiltriert. Zur weiteren Reinigung wird noch mehrmals mit Wasser gewaschen und am Hochvakuum getrocknet. Tabelle 2 zeigt die den 1,4-Diferrocenyldiazabutadienen Ia', $Ib_1$', $Ib_2$', Ic' entsprechenden Daten.

Tabelle 2:

| 1,4-Diferrocenyldiazabutadien | Aminoferrocen | Menge Aminoferrocen | Menge Glyoxal | Ausbeute |
|---|---|---|---|---|
| Ia' | IIIa | 0,86g (4,3 mmol) | 0,3g (2,1 mmol) | 0,86g, 94,8% |
| $Ib_1$' | $IIIb_1$ | 0,68g (2,2 mmol) | 0,16g (1,1 mmol) | 0,54g, 76,3% |
| $Ib_2$' | $IIIb_2$ | 0,827g (2,4 mmol) | 0,175g (1,2 mmol) | 0,64g, 60% |
| Ic' | IIIc | 38mg (0,14 mmol) | 10mg (0,07 mmol) | 38mg, 95,7% |

Daten für **Ia'**: violette Kristalle, m.p.: Zers. ab 200°C, $C_{22}H_{20}Fe_2N_2$, $^1H$-NMR($CD_2Cl_2$): δ 4.19 (s, 10H, $cp_{unsubst}$), 4.38 (m, 4H, $cp_{subst}$), 4.63 (m, 4H, $cp_{subst}$), 8.33 (s, 2H, Imin).

Daten für **$Ib_1$'**: violette Kristalle, m.p.: Zers. ab 200°, $C_{28}H_{36}Fe_2N_2Si_2$, $^1H$-NMR($CDCl_3$): δ 0.20 (s, 18H, $SiMe_3$), 4.10 (m, 4H, $cp_{subst}$), 4.34 (m, 4H, $cp_{subst}$), 4.36 (m, 4H, $cp_{subst}$), 4.59 (m, 4H, $cp_{subst}$), 8.32 (s, 2H, Imin).

Daten für **$Ib_2$'**: violette Kristalle, m.p.: Zers. ab 200°, $C_{38}H_{52}Fe_2N_2Si_2$, $^1H$-NMR($CDCl_3$): δ 0.18 (s, 6H, $SiMe_2$), 0.50-2.00 (m, 11H, cHex), 4.05 (m, 4H, $cp_{subst}$), 4.34 (m, 4H, $cp_{subst}$), 4.36 (m, 4H, $cp_{subst}$), 4.58 (m, 4H, $cp_{subst}$), 8.32 (s, 2H, Imin).

Daten für **Ic'**: violette Kristalle, $C_{32}H_{40}Fe_2N_2$, m.p.: Zers. ab 90°C, $^1H$-NMR($CD_2Cl_2$): δ 1,82 (s, 30H, 10 x $CH_3$), 4,18 (m, 4H, $C_{3,3',4,4'}$H), 4,21 (m, 4H, $C_{2,2',5,5'}$H), 7,55 (m, 1H, Imin), 7,70 (m, 1H, Imin).

Bis-(ferrocenylimino)-acenaphthen Ia":

0,467g (2,32 mmol) Aminoferrocen **IIIa** werden zusammen mit 1,16 ml (2,32 mmol) Trimethylaluminium (2 molar in Toluol) in Analogie zu B.Bildstein, P. Denifl *Synthesis* (1994) 158 bei 70°C drei Stunden in 50 ml absolutem Toluol gerührt. Ohne Abzukühlen gibt man anschließend 0,105g (0,58 mmol) Acenaphthenchinon hinzu und rührt über

Nacht bei 110°C. Das Lösungsmittel wird im Hochvakuum abgezogen und die Bestandteile des braunroten Rückstandes durch Chromatographie ($Al_2O_3$ basisch, Laufmittel: Petrolether/Ether = 1:1) getrennt.

Ausbeute: 0,230 mg (0,42 mmol, 72,3 % d. Theorie)

Daten für Bis-(ferrocenylimino)-acenaphthen **Ia"**: blaue Kristalle, $C_{32}H_{24}Cl_2CoFe_2N_2$, m.p.: Zers. ab 150°C, $^1$H-NMR($CD_2Cl_2$): δ 4.23-4.53 (m, 18H, Ferrocenyl), 7.39-7.90 (m, 6H, Acenaphthen-H). Herstellung der Metallkomplexe (allg. Arbeitsvorschrift):

Ein Moläquivalent Ligand wird in Tetrahydrofuran oder Methylenchlorid mit einem Moläquivalent des jeweiligen Metallsalzes ($NiCl_2$(DME), $NiBr_2(DME)_2$, $CoCl_2$, $PdCl_2(Benzonitril)_2$, Pd(COD)ClMe) bei Raumtemperatur zusammengegeben und zwei Stunden gerührt. Das Lösungsmittel wird am Rotationsverdampfer abgezogen, der grüne Feststoff mit Ether ausgewaschen, abfiltriert und mit Ether gewaschen. Im Falle des Pd(COD)ClMe muß noch mit Hexan digeriert werden, um einen amorphen, dunkelgrünen Feststoff zu erhalten. Tabelle 3 zeigt die den Metallkomplexen VIIa'$_a$, VIIa'$_b$, VIIa'$_c$, VIIa'$_d$, VIIb$_1$'$_a$, VIIb$_1$'$_b$, VIb$_1$'$_e$, VIIb$_1$'$_d$, VIIa"$_a$, VIIa"$_b$, VIIa"$_c$, VIIa"$_d$ entsprechenden Daten.

Tabelle 3

| Metallkomplex | Diazabutadien | Metallsalz ($MX_2$) | Ausbeute |
|---|---|---|---|
| VIIa'$_a$ | Ia' | $PdCl_2$ | 64,0% |
| VIIa'$_b$ | Ia' | $Pd(Cl)CH_3$ | 80,0% |
| VIIa'$_c$ | Ia' | $NiCl_2$ | 98,0% |
| VIIa'$_d$ | Ia' | $CoCl_2$ | 82,5% |
| VIIb$_1$'$_a$ | Ib$_1$' | $PdCl_2$ | 81,4% |
| VIIb$_1$'$_b$ | Ib$_1$' | $Pd(Cl)CH_3$ | 60,7% |
| VIIb$_1$'$_e$ | Ib$_1$' | $NiBr_2$ | 66,0% |
| VIIb$_1$'$_d$ | Ib$_1$' | $CoCl_2$ | 68,1% |
| VIIa"$_a$ | Ia" | $PdCl_2$ | 91,3% |
| VIIa"$_b$ | Ia" | $Pd(Cl)CH_3$ | 70,0% |
| VIIa"$_c$ | Ia" | $NiCl_2$ | 85,8% |
| VIIa"$_d$ | Ia" | $CoCl_2$ | 77,2% |

Daten für **VIIa'$_a$**: grüne Kristalle, $C_{22}H_{20}Cl_2CoFe_2N_2Pd$, m.p.: <300°C, IR(KBr): (cm$^{-1}$) 3091w, 1624m, 1530s, 1425s, 1412m, 1375w, 1350w, 1238vs, 1169w, 1107s, 1032m, 1001m, 926w, 827s, 642w, 474s.

Daten für **VIIa'$_b$**: grüne Kristalle, $C_{23}H_{23}ClFe_2N_2Pd$, m.p.: Zers. ab 200°C, IR(KBr): (cm$^{-1}$) 3083w, 2963w, 2886w, 1634m, 15741m, 1429m, 1410m, 1375m, 1348m, 1234w, 1165w, 1107s, 1026s, 1001s, 924s, 889w, 818vs, 644w, 542w, 490vs, 470vs. MS(FAB): m/z(%) 582(50) ($M^+$), 530(100) ($M^+$-$CH_3Cl$). UV($CH_2Cl_2$) $\lambda_{max}(\varepsilon)$ = 371nm (11000), 729nm (4000).

Daten für **VIIa'$_c$**: grüne Kristalle, $C_{22}H_{20}Cl_2Fe_2NiN_2$, m.p.: <300°C, IR(KBr): (cm$^{-1}$) 3083w, 1628m, 1574vs, 1472m, 1441s, 1410m, 1375w, 1369w, 1259m, 1105m, 1051w, 1038w, 1024w, 1001m, 960w, 874s, 818w, 798w, 520w, 486s, 461w. MS(FAB): m/z(%) 517(100) ($M^+$-Cl), 482(50) ($M^+$-2Cl). UV($CH_2Cl_2$) $\lambda_{max}(\varepsilon)$ = 411nm (17000), 780nm (8000).

Daten für **VIIa'$_d$**: grüne Kristalle, $C_{22}H_{20}Cl_2CoFe_2N_2$, m.p.: <300°C; IR(KBr): (cm$^{-1}$) 3087w, 1645m, 1574vs, 1474w, 1439s, 1410m, 1385s, 1375m, 1259m, 1105m, 1049m, 1038m, 1020m, 1001s, 960w, 818vs, 798w, 648w, 519m, 493s, 486m, 461w. MS(FAB): m/z(%) 518(100) ($M^+$-Cl). UV($CH_2Cl_2$) $\lambda_{max}(\varepsilon)$ = 416nm (25000), 830nm (13000).

Daten für **VIIb$_1$'$_a$**: grüne Kristalle, $C_{28}H_{36}Cl_2Fe_2N_2Si_2Pd$, m.p.: Zers. ab 200°C; IR(KBr): (cm$^{-1}$) 3087w, 2954m, 2896w, 1624w, 1533m, 1427m, 1406w, 1375w, 1364w, 1302w, 1248s, 1163s, 1038s, 924w, 901m, 877w, 837vs, 754m, 692w, 630w, 482m, 418w. UV($CH_2Cl_2$) $\lambda_{max}(\varepsilon)$ = 398.5nm (12000), 846.5nm (5000).

Daten für **VIIb$_1$'$_b$**: grüne Kristalle, $C_{29}H_{39}ClFe_2N_2Si_2Pd$, m.p.: Zers. ab 200°C; IR(KBr): (cm$^{-1}$) 3087w, 2954m, 2894w, 1632w, 1541w, 1427w, 1404w, 1383w, 1373w, 1364w, 1248s, 1163s, 1095w, 1036m, 924w, 901m, 874w, 837vs, 754w, 692w, 630w, 495w. MS(FAB): m/z(%) 673(10) ($M^+$-$CH_3$,Cl), 568(100) ($M^+$-$PdCH_3Cl$). UV($CH_2Cl_2$) $\lambda_{max}$ = 376nm (10000), 731nm (4000).

Daten für **VIIb$_1$'$_e$**: grüne Kristalle, $C_{28}H_{36}Br_2Fe_2N_2Si_2Ni$, m.p.: Zers. ab 200°C; IR(KBr): (cm$^{-1}$) 3077w, 2956m, 1634w, 1564s, 1470w, 1437m, 1375w, 1364w, 1250s, 1165s, 1053w, 1036m, 960m, 901m, 876w, 837vs, 818s, 754w, 630w, 522m, 492w, 476w. MS(FAB): m/z(%) 787(20) ($M^+$), 707(100) ($M^+$-Br), 626(50) ($M^+$-2Br), 548(70) ($M^+$-$NiBr_2$). UV($CH_2Cl_2$) $\lambda_{max}(\varepsilon)$ = 421nm (13000), 807nm (6000).

Daten für **VIIb$_1$'$_d$**: grüne Kristalle, $C_{28}H_{36}CoCl_2Fe_2N_2Si_2$, m.p.: Zers.ab 200°C, IR(KBr): ($cm^{-1}$) 2956m, 1628w, 1568s, 1472w, 1439m, 1375w, 1364w, 1250s, 1163s,1051w, 1038m), 960w, 901m, 877m, 837vs, 754w, 646w, 630w, 520w, 497w, 474w. MS(FAB): m/z(%) 697(7) ($M^+$), 662(40) ($M^+$-Cl), 627(7) ($M^+$-2Cl), 568(100) ($M^+$-$CoCl_2$). UV($CH_2Cl_2$) $\lambda_{max}(\varepsilon)$ = 422.5nm (11000), 846nm (6000).

Daten für **VIIa''$_a$**: grüne Kristalle, $C_{32}H_{24}Cl_2Fe_2N_2Pd$, m.p.: Zers. ab 200°C, IR (KBr) ($cm^{-1}$) 1633s, 1612m, 1430w, 1262w, 1110m, 1082w, 1042w, 863m, 764m, MS (FAB) m/z (%) = 691 (8) ($M^+$-Cl), 653 (15) ($M^+$-2 Cl), 549 (14) ($M^+$-$PdCl_2$). UV($CH_2Cl_2$) $\lambda_{max}(\varepsilon)$ = 312nm (22000), 331nm (22000), 333nm (22000), 836nm (5000).

Daten für **VIIa''$_b$**: grüne Kristalle $C_{33}H_{27}ClFe_2N_2Pd$, m.p.: Zers.ab 170°C, IR (KBr) ($cm^{-1}$)1719m, 1656m, 1630s, 1432m, 1420m, 1281m, 1262m, 1127m, 1106s, 1055s, 1028s, 1001s, 828 s, 780s. MS(FAB) m/z (%) = 706 (23) ($M^+$+H), 669 (5) ($M^+$-Cl), 653 (80) ($M^+$-Cl,-Me), 548 (68) ($M^+$-PdClMe). UV($CH_2Cl_2$) $\lambda_{max}(\varepsilon)$ = 338nm (13000), 684nm (2000).

Daten für **VIIa''$_c$**: grüne Kristalle, $C_{32}H_{24}Cl_2Fe_2N_2Ni$, m.p.: Zers. ab 150°C, IR (KBr) ($cm^{-1}$)1630s, 1432w, 1262w, 1105m, 1042m, 1020m, 842m, 81 m. MS(FAB) m/z(%) 641 (22) ($M^+$-Cl), 606 (31) ($M^{\bullet+}$-2 Cl), 549 (18) ($M^+$-$NiCl_2$). UV($CH_2Cl_2$) $\lambda_{max}(\varepsilon)$ = 333nm (7000), 729nm (2000).

Daten für **VIIa''$_d$**: grüne Kristalle, $C_{32}H_{24}Cl_2CoFe_2N_2$, m.p.: Zers. ab 200°C, IR (KBr) ($cm^{-1}$)1633s, 1430w, 1262w, 1110m, 1042w, 863m. MS (FAB) m/z(%) = 677 (4) ($M^+$), 642 (5) ($M^+$-Cl), 549 (4) ($M^+$-$CoCl_2$). UV($CH_2Cl_2$) $\lambda_{max}$ ($\varepsilon$) = 311nm (7000), 345nm (7000), 779nm (2000).

Figuren 1 bis 5 zeigen entsprechende Reaktionsschemata zur Herstellung der aufgeführten Verbindungen.

Homopolymerisation von Ethen

Aktivierung mit MAO (Methylaluminoxan), Polymerisationsbeispiel 1

140 ml trocknes Toluol wird in 250 ml Vierhalsglaskolben vorgelegt. Nach der Zugabe von 12,4 ml (19 mmol) MAO und 13,8 mg (19 µmol) des **Katalysators VIIb$_1$'$_c$** wird Ethen mit einem Durchsatz 40 l/h durch die Lösung drucklos durchgeblasen. Die Polymerisationstemperatur wird auf 50-55 °C eingestellt. Nach 4,5 h wird die Polymerisation durch Zugabe von HCl/MeOH abgebrochen. Die Mischung wird im Scheidetrichter getrennt, und die organische (Toluol-) Phase wird mit $H_2O$ gewaschen und getrocknet. Nach Filtration durch eine Säule mit Aluminiumoxid (neutral) wird das Polymer durch Verdampfung des Toluols (75 °C, 0,1 mbar, 3 h) abgetrennt. Tabelle 4, Polymerisationsbeispiel 1 faßt die Daten des erhaltenen Polyethylens zusammen.

Kationische Aktivierung, Polymerisationsbeispiel 2

140 ml trocknes Toluol wird in 250 ml Vierhalsglaskolben vorgelegt. Nach der Zugabe von 1,5 ml (3 mmol) TIBAL (Triisobutylaluminium) und 220 mg (0,3 mmol) des Katalysators VIIb$_1$'$_b$ wird die Reaktionsmischung 1 h gerührt. Danach werden 268 mg (ca. 0,333 mmol) N,N-Dimethylanilinium-tetrakis(pentafluorophenyl)-borat (DMAB) zugegeben, und es wird weitere 30 min gerührt. Danach wird Ethen mit einem Durchsatz 40 l/h durch die Lösung drucklos durchgeblasen. Die Polymerisationstemperatur wird auf 50-55 °C eingestellt. Nach 5 h wird die Polymerisation durch Zugabe von HCl/MeOH abgerbrochen. Die Mischung wird im Scheidetrichter getrennt. Die organische (Toluol-)Phase wird mit $H_2O$ gewaschen und getrocknet. Nach Filtration durch eine Säule mit Aluminiumoxid (neutral) wird das Polymer durch Verdampfung des Toluols (75 °C, 0,1 mbar, 3 h) abgetrennt. Tabelle 4, Polymerisationsbeispiel 2 faßt die Daten des erhaltenen Polyethylens zusammen.

Tabelle 4

| **Polymerisationsbeispiel** | **Katalysator** | **Aktivator** | **Polymerisationstemperatur, °C** | η1) |
|---|---|---|---|---|
| 1 | VIIb$_1$'$_c$ | MAO | 50-55 | 0,4 |
| 2 | VIIb$_1$'$_b$ | DMAB | 50-55 | 0,3 |

1)h wurde gemäß ISO 1628-3 ermittelt

**Patentansprüche**

1. Verfahren zur Herstellung von 1,2-Diimin-Verbindungen der allgemeinen Formel I

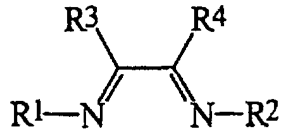 I

in der die Symbole die folgende Bedeutung haben

$R^1$ und $R^2$ unabhängig voneinander Alkyl-, Aryl- oder Metallocenyl-Reste,

und

$R^3$, $R^4$ unabhängig voneinander H, Alkyl- oder Arylreste,

oder

$R^3$ und $R^4$ bilden gemeinsam einen cyclischen Rest, so daß, einschließlich der beiden Imin-Kohlenstoffatome, ein 5- bis 8-gliedriger Ring gebildet wird, der gesättigt oder ungesättigt und unsubstituiert oder beliebig mit Kohlenwasserstoffresten substituiert sein kann,

durch Umsetzung von 1,2-Dicarbonylverbindungen mit primären Aminen, **dadurch gekennzeichnet, daß** die Amine vor der Umsetzung mit den 1,2-Dicarbonylverbindungen mit Trialkylaluminium-Verbindungen aktiviert werden.

**2.** Verfahren nach Anspruch 1, **dadurch gekennzeichnet, daß** die Reste $R^1$ und $R^2$ unabhängig voneinander substituierte Aryl- oder Metallocenylreste sind.

**3.** Verfahren nach Anspruch 2, **dadurch gekennzeichnet, daß** $R^1$ und $R^2$ unabhängig voneinander Metallocenylreste sind.

**4.** Verfahren nach Anspruch 3, **dadurch gekennzeichnet, daß** $R^1$ und $R^2$ Ferrocenylreste sind.

**5.** Verfahren nach Anspruch 4, **dadurch gekennzeichnet, daß** ein Ferrocenylrest der allgemeinen Formeln IIa, IIb oder IIc eingesetzt wird,

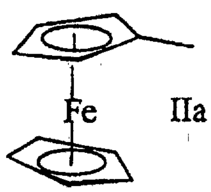 IIa 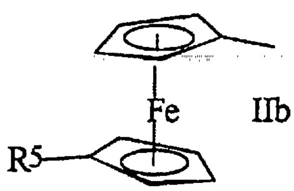 IIb 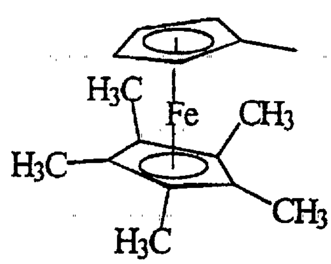 IIc

in denen $R^5$ -Me, $-SiMe_3$ oder $-SicHexMe_2$ ist.

**6.** Verfahren nach einem der Ansprüche 1 bis 5, **dadurch gekennzeichnet, daß** 1,2-Dicarbonylverbindungen der allgemeinen Formel IV eingesetzt werden,

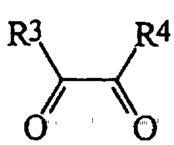 IV

in der die Symbole die folgende Bedeutung haben

$R^3$ und $R^4$ unabhängig voneinander H, Alkyl- oder Arylreste,

oder

$R^3$ und $R^4$ bilden gemeinsam einen cyclischen Rest, so daß, einschließlich der beiden Carbonylkohlenstoffatome, ein 5- bis 8-gliedriger Ring gebildet wird, der gesättigt oder ungesättigt und unsubstituiert oder beliebig mit Kohlenwasserstoffresten substituiert sein kann.

**7.** Verfahren nach Anspruch 6, **dadurch gekennzeichnet, daß** 1,2-Dicarbonylverbindungen der allgemeinen Formeln IVa oder IVb eingesetzt werden,

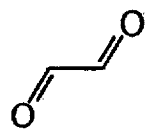
IVa

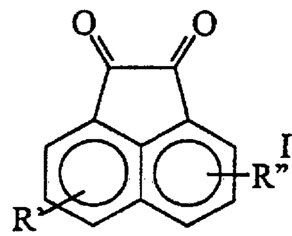
IVb

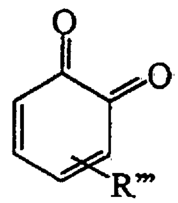
IVc

in denen R', R'', R''' H, Alkyl oder Aryl bedeuten.

**8.** Verfahren nach einem der Ansprüche 1 bis 7, **dadurch gekennzeichnet, daß** Trialkylaluminium-Verbindungen der allgemeinen Formel V eingesetzt werden,

$$R^6R^7R^8Al \qquad V$$

in der, $R^6$, $R^7$ und $R^8$ unabhängig voneinander $C_1$- bis $C_{10}$-Alkyl bedeuten.

**9.** Verfahren nach Anspruch 8, **dadurch gekennzeichnet, daß** $R^6$, $R^7$ und $R^8$ Methyl bedeuten.

**10.** Verwendung von Trialkylaluminium-Verbindungen zur Herstellung von 1,2-Diimin-Verbindungen aus 1,2-Dicarbonylverbindungen und primären Aminen.

**11.** 1,2-Diimin-Verbindungen der allgemeinen Formel I gemäß Anspruch 1, **dadurch gekennzeichnet, daß** $R^1$ und $R^2$ unabhängig voneinander Metallocenylreste sind.

**12.** Verbindungen nach Anspruch 11, **dadurch gekennzeichnet, daß** $R^1$ und $R^2$ Ferrocenylreste sind.

**13.** Verbindungen der allgemeinen Formel VII,

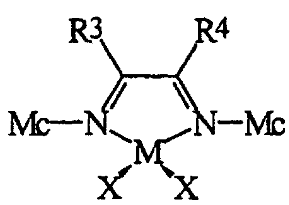
VII

worin die Symbole die folgende Bedeutung haben

$R^3$ und $R^4$ unabhängig voneinander H, Alkyl- oder Arylreste,

oder

$R^3$ und $R^4$ bilden gemeinsam einen cyclischen Rest, so daß, einschließlich der beiden Imin-Kohlenstoffatome, ein 5- bis 8-gliedriger Ring gebildet wird, der gesättigt oder ungesättigt und unsubstituiert oder beliebig mit Kohlenwasserstoffresten substituiert sein kann, und

Mc ein am Cyclopentadienylrest unsubstituierter oder substituierter Metallocenylrest,

M Übergangsmetall der VIII. Nebengruppe des Periodensystems der Elemente,

und

X ein Halogenid oder ein $C_1$- bis $C_{20}$-Alkylrest.

**14.** Verfahren zur Herstellung von Verbindungen der allgemeinen Formel VII gemäß Anspruch 13, durch Umsetzung von entsprechenden Verbindungen der allgemeinen Formel I mit Übergangsmetallsalzen von Metallen der VIII. Nebengruppe des Periodensystems der Elemente.

**15.** Verwendung von Verbindungen der allgemeinen Formel VII gemäß Anspruch 13 als Katalysatoren in einem verfahren zur Polymerisation ungesättigter Verbindungen.

**16.** Verfahren zur Herstellung von Pölyorefinen durch Polymerisation ungesättigter Verbindungen in Anwesenheit eines Aktivators und einer Verbindung der allgemeinen Formel VII gemäß Anspruch 13 als Katalysator.

**17.** Verfahren nach Anspruch 16, **dadurch gekennzeichnet, daß** Methylaluminoxan oder N, N-Dimethylanilinium-tetrakis(pentafluorophenyl)borat als Aktivator eingesetzt werden.

**18.** Verfahren nach Anspruch 16 oder 17, **dadurch gekennzeichnet, daß** eine ungesättigte Verbindung oder eine Kombination ungesättigter Verbindungen ausgewählt aus Ethylen, $C_3$- bis $C_{20}$-Monoolefinen, Ethylen und $C_3$- bis $C_{20}$-Monoolefinen, Cycloolefinen, Cycloolefinen und Ethylen und Cycloolefinen und Propylen eingesetzt wird.

**19.** Verfahren nach Anspruch 16 oder 17, **dadurch gekennzeichnet, daß** bei Einsatz eines Katalysators der allgemeinen Formel VII gemäß Anspruch 13 mit Pd(II) als Zentralmetall M ungesättigte verbindungen oder Kombinationen ungesättigter Verbindungen ausgewählt aus Ethylen, $C_3$- bis $C_{20}$-Monoolefinen, Ethylen und $C_3$- bis $C_{20}$-Monoolefinen, Ethylen und einem Alkylacrylat, Ethylen und Acrylsäure, Ethylen und Kohlenmonoxid, Ethylen, Kohlenmonoxid und einem Acrylatester oder einer Acrylsäure. Propylen und einem Alkylacrylat, Cycloolefinen, Cycloolefinen und Ethylen und Cycloolefinen und Propylen eingesetzt werden.

**20.** Verfahren nach einem der Ansprüche 16 bis 19, **dadurch gekennzeichnet, daß** Ethylen als ungesättigte Verbindung eingesetzt wird.

**21.** Verfahren nach einem der Ansprüche 16 bis 20, **dadurch gekennzeichnet, daß** die Polymerisation bei Temperaturen von 0 bis 100°C durchgeführt wird.

**22.** Verfahren nach einem der Ansprüche 16 bis 21, **dadurch gekennzeichnet, daß** die Polymerisation bei einem Druck von 0,1 bis 3000 bar durchgeführt wird.

**23.** Polyolefin, herstellbar in einem Verfahren nach einem der Ansprüche 16 bis 22.

## Claims

**1.** A process for preparing 1,2-diimine compounds of the formula I

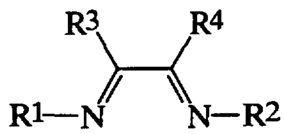

I

where the symbols have the following meanings

$R^1$ and $R^2$ are, independently of one another, alkyl, aryl or metallocenyl radicals,

and

$R^3$, $R^4$ are, independently of one another, H, alkyl or aryl radicals,

or

$R^3$ and $R^4$ are joined so as to form, with inclusion of the two imine carbon atoms, a 5- to 8-membered ring which may be saturated or unsaturated and may be unsubstituted or substituted by any hydrocarbon radicals,

by reacting 1,2-dicarbonyl compounds with primary amines, which have been activated with trialkylaluminum compounds prior to the reaction with the 1,2-dicarbonyl compounds.

**2.** A process as claimed in claim 1, wherein the radicals $R^1$ and $R^2$ are, independently of one another, substituted aryl radicals or metallocenyl radicals.

**3.** A process as claimed in claim 2, wherein $R^1$ and $R^2$ are, independently of one another, metallocenyl radicals.

**4.** A process as claimed in claim 3, wherein $R^1$ and $R^2$ are ferrocenyl radicals.

**5.** A process as claimed in claim 4, wherein a ferrocenyl radical of the formula IIa, IIb or IIc,

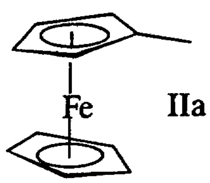

IIa

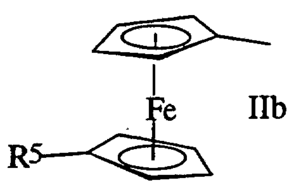

IIb

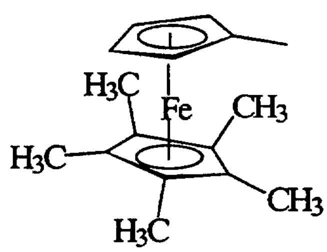

IIc

where $R^5$ is -Me, $-SiMe_3$ or $-SicHexMe_2$
is used.

**6.** A process as claimed in any of claims 1 to 5, wherein 1,2-dicarbonyl compounds of the formula IV,

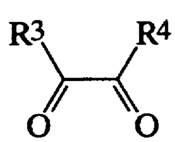

IV

where the symbols have the following meanings:

$R^3$ and $R^4$ are, independently of one another, H, alkyl or aryl radicals,

or

$R^3$ and $R^4$ are joined so as to form, with inclusion of the two carbonyl carbon atoms, a 5- to 8-membered ring which may be saturated or unsaturated and may be unsubstituted or substituted by any hydrocarbon radicals,
are used.

**7.** A process as claimed in claim 6, wherein 1,2-dicarbonyl compounds of the formula IVa or IVb,

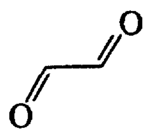 IVa 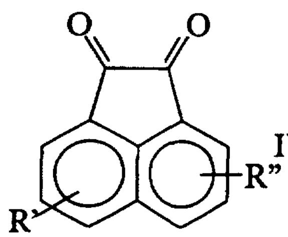 IVb 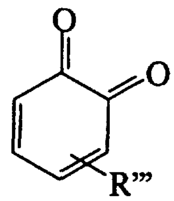 IVc

where R', R'', R''' are each H, alkyl or aryl,
are used.

**8.** A process as claimed in any of claims 1 to 7, wherein trialkylaluminum compounds of the formula V,

$$R^6R^7R^8Al \qquad V$$

where $R^6$, $R^7$ and $R^8$ are each, independently of one another, $C_1$-$C_{10}$-alkyl,
are used.

**9.** A process as claimed in claim 8, wherein $R^6$, $R^7$ and $R^8$ are each methyl.

**10.** The use of trialkylaluminum compounds for preparing 1,2-diimine compounds from 1,2-dicarbonyl compounds and primary amines.

**11.** A 1,2-diimine compound of the formula I as claimed in claim 1, wherein $R^1$ and $R^2$ are, independently of one another, metallocenyl radicals.

**12.** A compound as claimed in claim 11, wherein $R^1$ and $R^2$ are ferrocenyl radicals.

**13.** A compound of the formula VII,

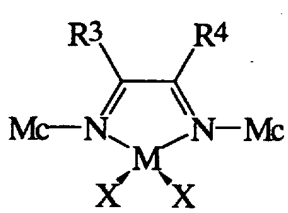 VII

where the symbols have the following meanings:

$R^3$ and $R^4$ are, independently of one another, H, alkyl or aryl radicals,

or

$R^3$ and $R^4$ are joined so as to form, with inclusion of the two imine carbon atoms, a 5- to 8-membered ring which may be saturated or unsaturated and may be unsubstituted or substituted by any hydrocarbon radicals, and

Mc is a metallocenyl radical which may be unsubstituted or substituted on the cyclopentadienyl radical,

M is a transition metal of transition group VIII of the Periodic Table of the Elements,

and

X is a halide or a $C_1$-$C_{20}$-alkyl radical.

**14.** A process for preparing compounds of the formula VII as claimed in claim 13, by reacting corresponding compounds of the formula I with salts of metals of transition group VIII of the Periodic Table of the Elements.

**15.** The use of compounds of the formula VII as claimed in claim 13 as catalysts in a process for the polymerization of unsaturated compounds.

**16.** A process for preparing polyolefins by polymerization of unsaturated compounds in the presence of an activator and a compound of the formula VII as claimed in claim 13 as catalyst.

**17.** A process as claimed in claim 16, wherein methylaluminoxane or N,N-dimethylanilinium tetrakis(pentafluorophenyl)borate is used as activator.

**18.** A process as claimed in claim 16 or 17, wherein an unsaturated compound or a combination of unsaturated compounds selected from the group consisting of ethylene, $C_3$-$C_{20}$-monoolefins, ethylene and $C_3$-$C_{20}$-monoolefins, cycloolefins, cycloolefins and ethylene and cycloolefins and propylene is used.

**19.** A process as claimed in claim 16 or 17, wherein, when using a catalyst of the formula VII as claimed in claim 13 with Pd(II) as central metal M, unsaturated compounds or combinations of unsaturated compounds selected from the group consisting of ethylene, $C_3$-$C_{20}$-monoolefins, ethylene and $C_3$-$C_{20}$-monoolefins, ethylene and an alkyl acrylate, ethylene and acrylic acid, ethylene and carbon monoxide, ethylene, carbon monoxide and an acrylate ester or an acrylic acid, propylene and an alkyl acrylate, cycloolefins, cycloolefins and ethylene and cycloolefins and propylene are used.

**20.** A process as claimed in any of claims 16 to 19, wherein ethylene is used as unsaturated compound.

**21.** A process as claimed in any of claims 16 to 20, wherein the polymerization is carried out at from 0 to 100°C.

**22.** A process as claimed in any of claims 16 to 21, wherein the polymerization is carried out at a pressure of from 0.1 to 3 000 bar.

**23.** A polyolefin which can be prepared by a process as claimed in any of claims 16 to 22.

**Revendications**

**1.** Procédé pour la préparation de composés de 1,2-diimine de formule générale I

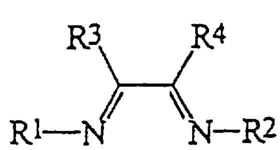 I

dans laquelle les symboles ont la signification suivante

$R^1$ et $R^2$ indépendamment l'un de l'autre, des restes alkyle, aryle ou métallocényle,

et

$R^3$, $R^4$ indépendamment l'un de l'autre, des restes H, alkyle ou aryle,

ou

$R^3$ et $R^4$ forment ensemble un reste cyclique, de manière à former conjointement avec les deux atomes de carbone d'imine un cycle ayant 5 à 8 chaînons, qui peut être saturé ou insaturé et non-substitué ou substitué à convenance avec des restes hydrocarbonés,

par réaction de composés 1,2-dicarbonylés avec des amines primaires, **caractérisé par le fait qu'**on active les amines avec la réaction avec les composés 1,2-dicarbonylés, avec des composés de trialkylaluminium.

2. Procédé selon la revendication 1, **caractérisé par le fait que** les restes $R^1$ et $R^2$ sont, indépendamment l'un de l'autre, des restes aryle ou métallocényle substitués.

3. Procédé selon la revendication 2, **caractérisé par le fait que** $R^1$ et $R^2$ sont, indépendamment l'un de l'autre, des restes métallocényle.

4. Procédé selon la revendication 3, **caractérisé par le fait que** $R^1$ et $R^2$ sont des restes ferrocényle.

5. Procédé selon la revendication 4, **caractérisé par le fait qu'**on utilise un reste ferrocényle de formule générales IIa, IIa ou IIc

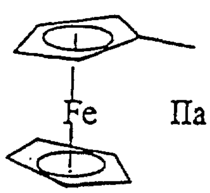
IIa

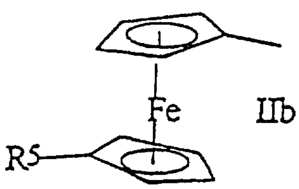
IIb

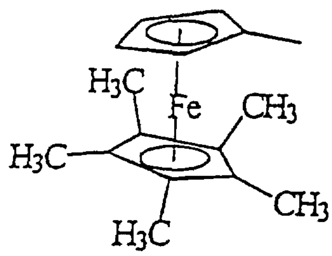
IIc

dans lesquelles $R^5$ est -Me, $-SiMe_3$ ou $-SicHexMe_2$.

6. Procédé selon l'une des revendications 1 à 5, **caractérisé par le fait qu'**on utilise des composés 1,2-dicarbonylés de formule générale IV,

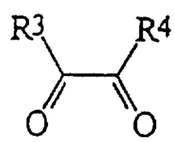
IV

dans laquelle les symboles ont la signification suivante

$R^3$, $R^4$ indépendamment l'un de l'autre, des restes H, alkyle ou aryle,

ou

$R^3$ et $R^4$ forment ensemble un reste cyclique, de manière à former conjointement avec les deux atomes de carbonyle un cycle ayant 5 à 8 chaînons, qui peut être saturé ou insaturé et non-substitué ou substitué à convenance avec des restes hydrocarbonés.

7. Procédé selon la revendication 6, **caractérisé par le fait qu'**on utilise des composés 1,2-dicarbonylés de formules générales IVa ou IVb,

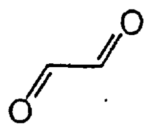 IVa 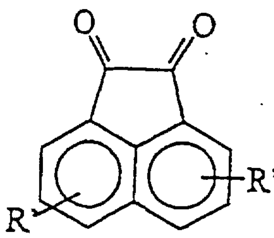 IVb 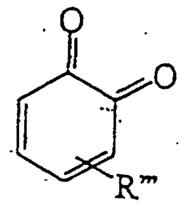 IVc

dans lesquelles R', R'', R''' désignent un reste H, alkyle ou aryle.

8. Procédé selon l'une des revendications 1 à 7, **caractérisé par le fait qu'**on utilise des composés de trialkylaluminium de formule générale V

$$R^6R^7R^8Al \qquad V$$

dans laquelle $R^6$, $R^7$ et $R^8$ désignent, indépendamment l'un de l'autre, des groupes alkyle en $C_1$ à $C_{10}$.

9. Procédé selon la revendication 8, **caractérisé par le fait que** $R^6$, $R^7$ et $R^8$ désignent le méthyle.

10. Utilisation de composés de trialkylaluminium pour la préparation de composés de 1,2-diimine à partir de composés de 1,2-dicarbonyle et d'amines primaires.

11. Composés de 1,2-diimine de formule générale I selon la revendication 1, **caractérisés par le fait que** $R^1$ et $R^2$ sont, indépendamment l'un de l'autre, des restes métallocényle.

12. Composés selon la revendication 11, **caractérisés par le fait que** $R^1$ et $R^2$ sont des restes ferrocényle.

13. Composés de formule générale VII,

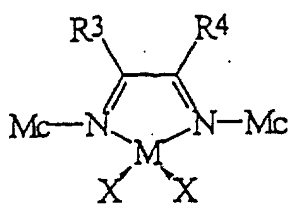 VII

dans laquelle les symboles ont la signification suivante

$R^3$, $R^4$ indépendamment l'un de l'autre, des restes H, alkyle ou aryle,

ou

$R^3$ et $R^4$ forment ensemble un reste cyclique, de manière à former conjointement avec les deux atomes de carbone de l'imine un cycle ayant 5 à 8 chaînons, qui peut être saturé ou insaturé et non-substitué ou substitué à convenance avec des restes hydrocarbonés, et

Mc un reste métallocényle non-substitué ou substitué sur le reste cyclopentadiényle,
M un métal de transition du sous-groupe VIII de la classification périodique des éléments,

et

X un halogénure ou un reste alkyle en $C_1$ à $C_{20}$.

**14.** Procédé pour la préparation de composés de formule générale VII selon la revendication 13, par réaction de composés correspondants de formule générale I avec des sels de métaux de transition de métaux du sous-groupe VIII de la classification périodique des éléments.

**15.** Utilisation de composés de formule générale VII selon la revendication 13 comme catalyseurs dans un procédé pour la polymérisation de composés insaturés.

**16.** Procédé pour la préparation de polyoléfines par polymérisation de composés insaturés en présence d'un activateur et d'un composé de formule générale VII selon la revendication 13 comme catalyseur.

**17.** Procédé selon la revendication 16, **caractérisé par le fait qu'**on utilise du méthylaluminoxane ou du tétrakis (pentafluorophényl)borate de N,N-diméthylaluminium comme activateur.

**18.** Procédé selon la revendication 16 ou 17, **caractérisé par le fait qu'**on utilise un composé insaturé ou une combinaison de composés insaturés choisis parmi l'éthylène, des monooléfines en $C_3$ à $C_{20}$, l'éthylène et des monooléfines en $C_3$ à $C_{20}$, des cyclooléfines, des cyclooléfines et l'éthylène, et des cyclooléfines et le propylène.

**19.** Procédé selon la revendication 16 ou 17, **caractérisé par le fait que** lors de l'utilisation d'un catalyseur de formule générale VII selon la revendication 13 avec Pd(II) comme métal central M, on utilise des composés insaturés ou des combinaisons de composés insaturés choisis parmi l'éthylène, des monooléfines en $C_3$ à $C_{20}$, l'éthylène et des monooléfines en $C_3$ à $C_{20}$, l'éthylène et un acrylate d'alkyle, l'éthylène et l'acide acrylique, l'éthylène et le monoxyde de carbone, l'éthylène, le monoxyde de carbone et un ester acrylate ou un acide acrylique, le propylène et un acrylate d'alkyle, des cyclooléfines, des cyclooléfines et l'éthylène et des cyclooléfines et le propylène.

**20.** Procédé selon l'une des revendications 16 à 19, **caractérisé par le fait qu'**on utilise de l'éthylène comme composé insaturé.

**21.** Procédé selon l'une des revendications 16 à 20, **caractérisé par le fait qu'**on effectue la polymérisation à des températures de 0 à 100°C.

**22.** Procédé selon l'une des revendications 16 à 21, **caractérisé par le fait qu'** la polymérisation est effectuée à une pression de 0,1 à 3000 bar.

**23.** Polyoléfine, pouvant être préparée dans un procédé selon l'une des revendications 16 à 22.

# FIG.1

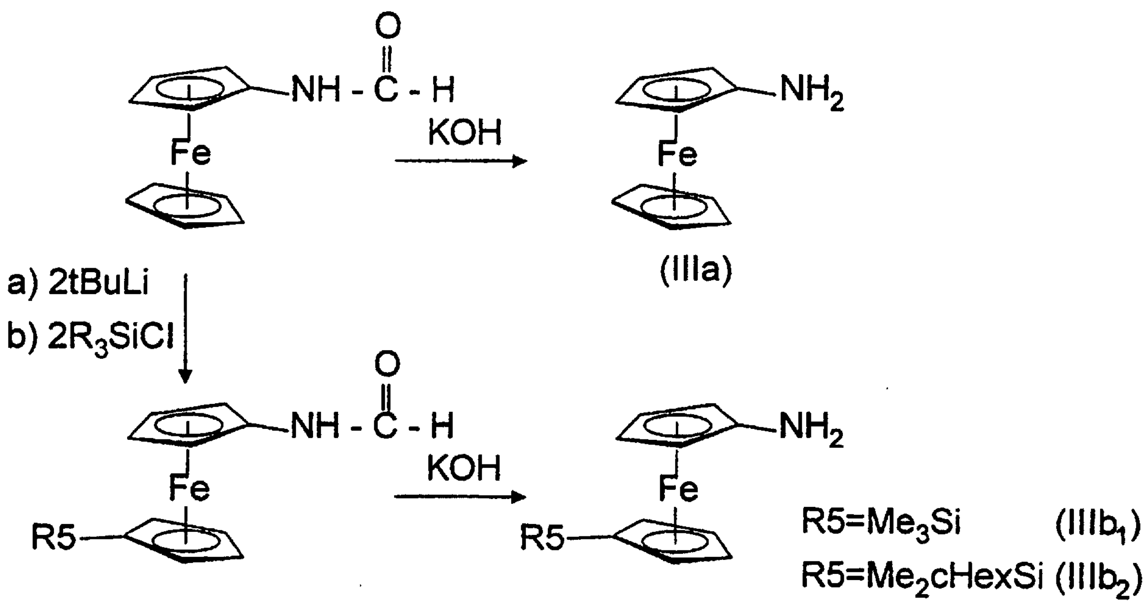


(IIIa)

$R5=Me_3Si$ ($IIIb_1$)

$R5=Me_2cHexSi$ ($IIIb_2$)

FIG.2

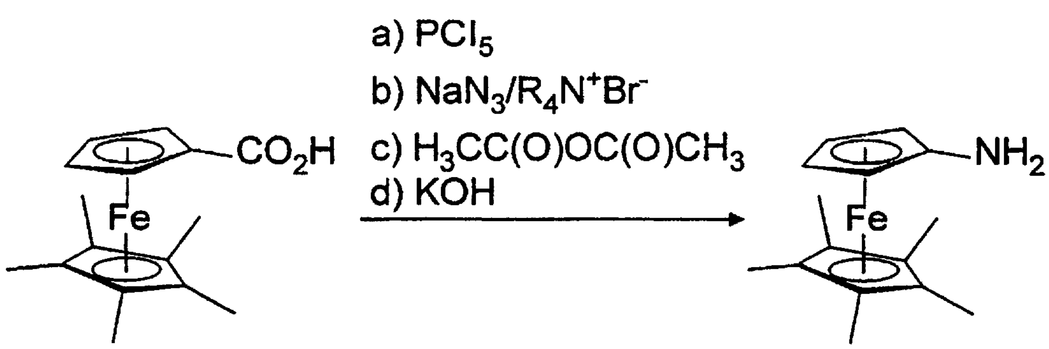


(IIIc)

# FIG.3

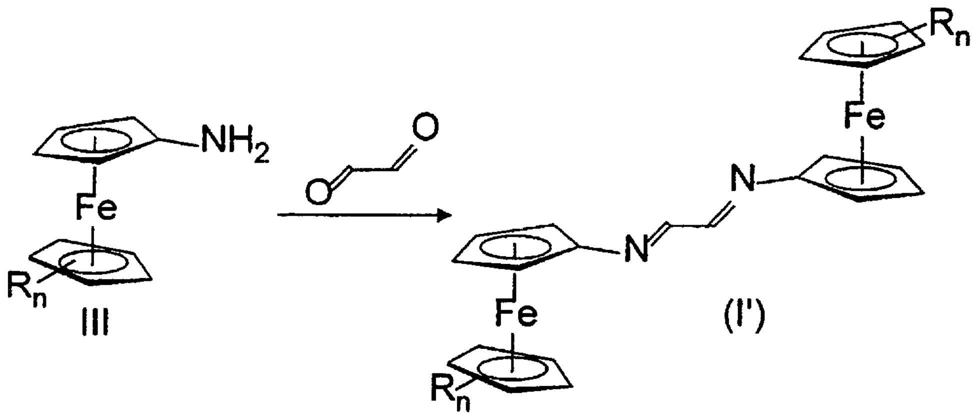

III

(I')

(IIIa) : $R_n$=H, n=1

($IIIb_1$): $R_n$=$Me_3Si$, n=1

(IIIb2): $R_n$=$Me_2cHexSi$, n=1

(IIIc) : $R_n$=Me, n=5

(Ia') : $R_n$=H, n=1

($Ib_1'$): $R_n$=$Me_3Si$, n=1

($Ib_2'$): $R_n$=$Me_2cHexSi$, n=1

(Ic') : $R_n$=Me, n=5

FIG.4

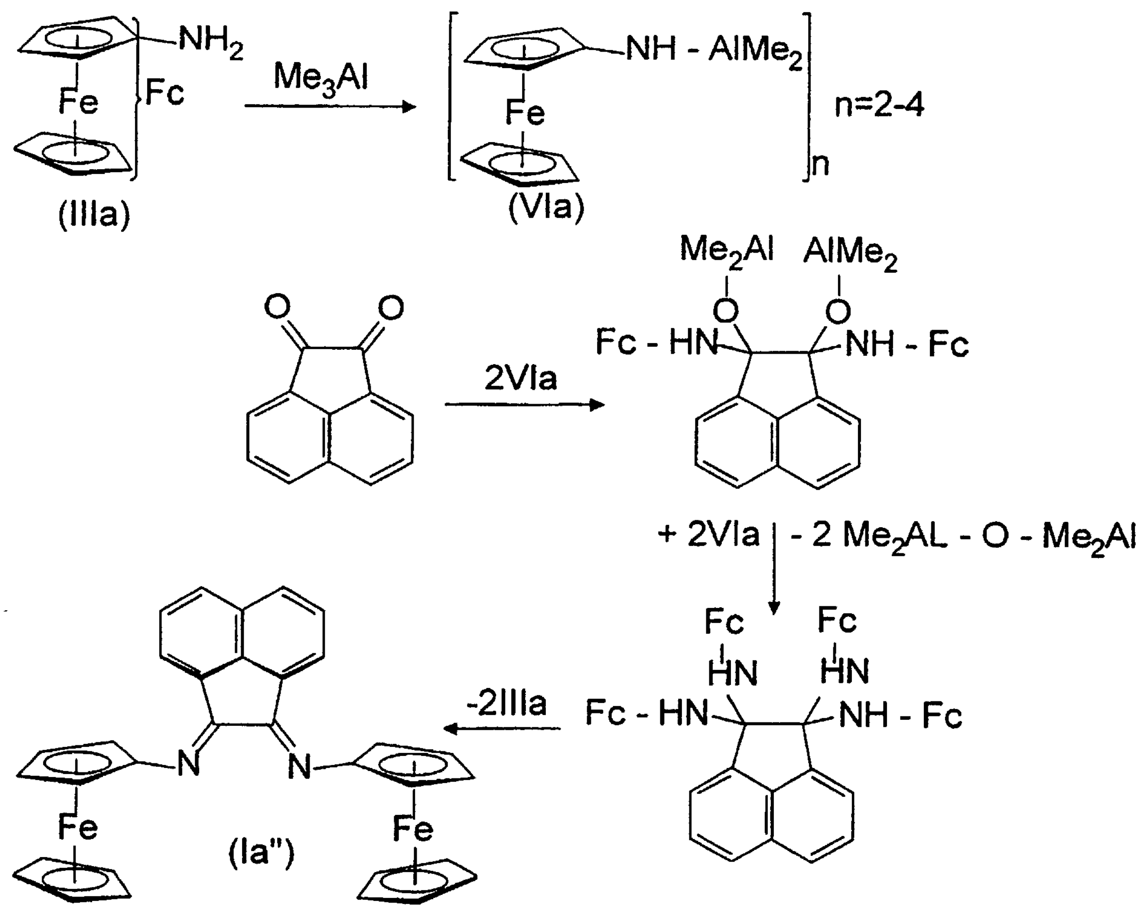


(IIIa) (VIa) (Ia")

# FIG.5

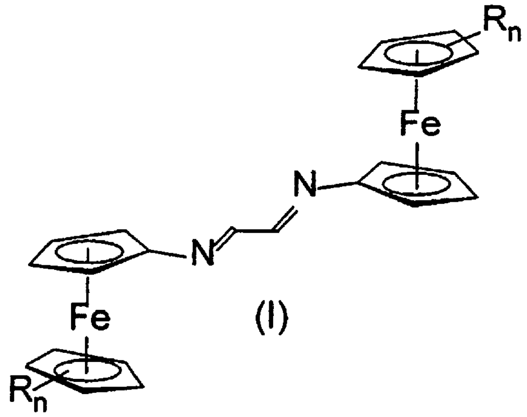

(I)

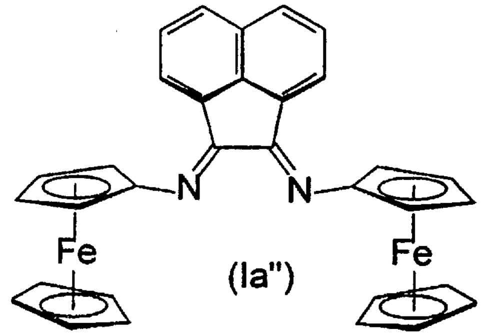

(Ia")

(Ia') : $R_n$=H, n=1
(Ib$_1$'): $R_n$=$Me_3Si$, n=1

$MX_2$

$MX_2$

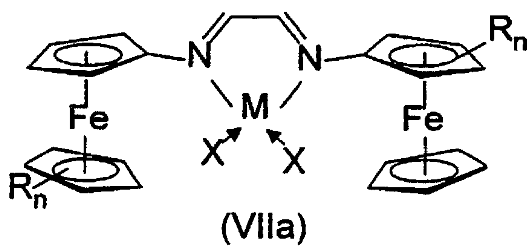

(VIIa)

(VIIa') : $R_n$=H, n=1
(VIIb$_1$'): $R_n$=$Me_3Si$, n=1

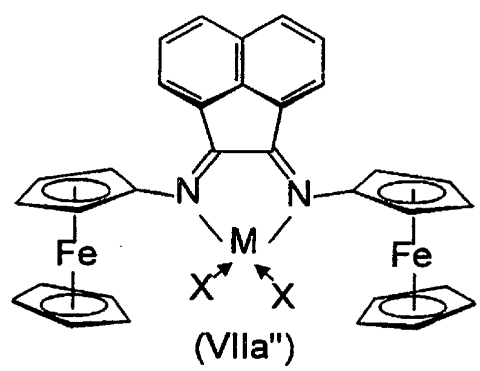

(VIIa")

| VII | I | $MX_2$ |
|---|---|---|
| VIIa'$_a$ | Ia' | $PdCl_2$ |
| VIIa'$_b$ | Ia' | $Pd(Cl)CH_3$ |
| VIIa'$_c$ | Ia' | $NiCl_2$ |
| VIIa'$_d$ | Ia' | $CoCl_2$ |
| VIIb$_1$'$_a$ | Ib$_1$' | $PdCl_2$ |
| VIIb$_1$'$_b$ | Ib$_1$' | $Pd(Cl)CH_3$ |
| VIIb$_1$'$_c$ | Ib$_1$' | $NiBr_2$ |
| VIIb$_1$'$_d$ | Ib$_1$' | $CoCl_2$ |

| VII | I | $MX_2$ |
|---|---|---|
| VIIa"$_a$ | Ia" | $PdCl_2$ |
| VIIa"$_b$ | Ia" | $Pd(Cl)CH_3$ |
| VIIa"$_c$ | Ia" | $NiCl_2$ |
| VIIa"$_d$ | Ia" | $CoCl_2$ |
| | | |
| | | |
| | | |
| | | |